# EUROPEAN PATENT APPLICATION

(11) **EP 1 749 823 A1**
(43) Date of publication of application: **07.02.2007**
(21) Application number: 05743280.9
(22) Date of filing: 27.05.2005
(51) Int. Cl.: C07D 241/38, C07D 249/18, C07D 271/12, C07D 285/14, C09K 11/06, H05B 33/14, H05B 33/22

(54) **AMINE COMPOUND AND ORGANIC ELECTROLUMINESCENT ELEMENT EMPLOYING THE SAME**

(30) Priority: 28.05.2004 JP 2004158560
(71) Applicant: IDEMITSU KOSAN CO., LTD., Tokyo 100-8321 (JP)
(72) Inventor: WATANABE, Masami, 2990293 (JP); HOSOKAWA, Chishio, 2990293 (JP); KONDO, Hirofumi, 2990293 (JP); HIRAO, Toshikazu, 6620084 (JP)
(74) Representative: Gille, Christian
(86) International application number: PCT/JP2005/009754
(87) International publication number: WO 2005/115995

(57) **Abstract**

An amine-based compound having a specific structure and an organic electroluminescence device which comprises at least one layer comprising at least a light emitting layer and is disposed between a cathode and an anode, wherein at least one layer in the organic thin film layer comprises the amine-based compound singly or as a component of a mixture. The organic electroluminescence device exhibits an excellent balance in physical properties such as a small ionization potential, a great band gap energy, a great efficiency of injection and a great mobility and excellent heat resistance and current efficiency due to the amine-based compound.

## Description

### TECHNICAL FIELD

The present invention relates to an amine compound (hereinafter, referred to as an "amine-based compound") and an organic electroluminescent (hereinafter, "electroluminescent" and "electroluminescence" will be referred to as "EL") element (hereinafter, an "EL element" will be referred to as an "EL device") employing the compound. More particularly, the present invention relates to an organic EL device exhibiting excellent heat resistance and efficiency of light emission and an amine-based compound enabling to obtain the device.

### BACKGROUND ART

An organic EL device is a spontaneous light emitting device which utilizes the principle that a fluorescent substance emits light by energy of recombination of holes injected from the anode and electrons injected from the cathode when an electric field is applied. The organic EL device exhibits excellent visibility due to the spontaneous light emission and excellent impact resistance due to the completely solid structure, and the application to light emitting elements in various display devices is attracting attention.

Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinolato)aluminum for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the hole injecting material used for the organic EL device, high molecular weight amine compounds disclosed in Patent Reference 1, triarylamine polymers disclosed in Patent Reference 2 and phenylenediamine derivatives disclosed in Patent Reference 3 have been known.

Injection of holes into the anode is facilitated since these compounds have small ionization potentials, and mobility of holes is greater than that of star-burst amine derivatives such as the compounds disclosed in Patent Reference 4. Therefore, these compounds are suitable as the hole injecting material.

Materials used for an organic EL device can be divided into low molecular weight organic materials used for preparing the device in accordance with the vacuum vapor deposition process and organic polymer materials used for preparing the device in accordance with a coating process such as the spin coating process and the ink-jet process using a solution of the materials dissolved in a solvent. It is not always necessary that the polymer material used above is a material having a great molecular weight, and various materials can be used without problems as long as the materials form the amorphous condition at the temperature of the use. As such materials, materials called low molecular weight amorphous materials are attracting attention.

Examples of the conventional low molecular weight hole injecting and transporting material include the following compounds: 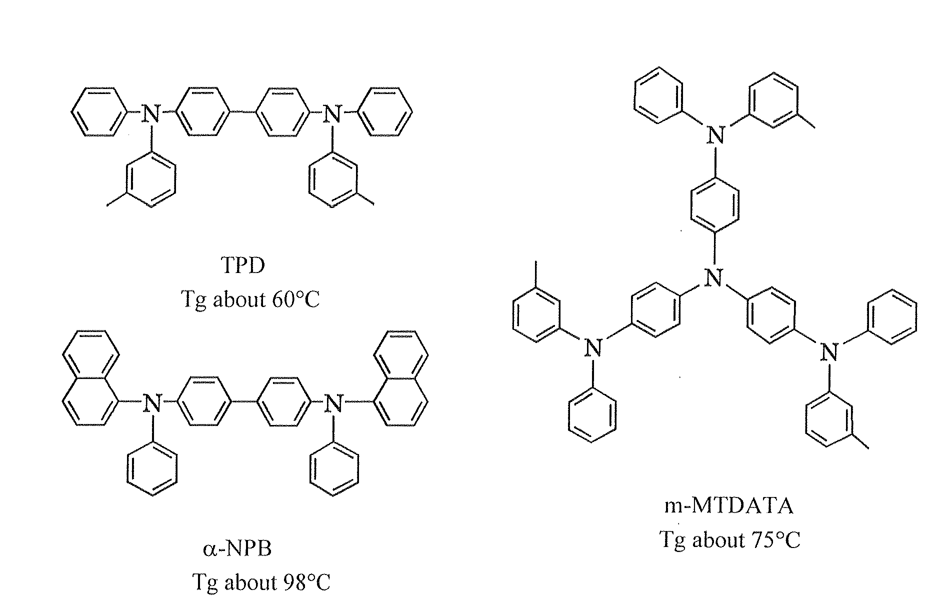 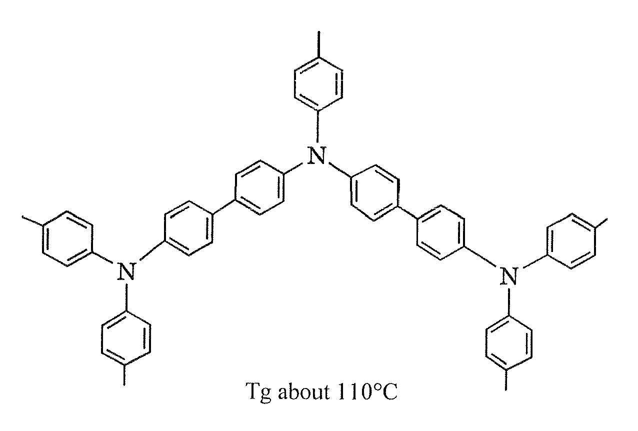 Among these compounds, TPD has been used widely since the compound has an ionization potential as small as 5.4 eV and exhibits a great mobility of holes. However, the compounds shown above as the examples exhibit poor heat resistance (low glass transition temperatures, Tg) and have a drawback in that crystallization takes place after a long time even at the room temperature, and the film becomes uneven.

As for the hole injecting and transporting polymers, the hole injecting property can be improved by doping electrically conductive polymers such as polythiophene and polyaniline with an acid such as PSS having the structure shown below. Hole injecting and transporting polymer into which TPD is introduced in the main chain or in side chains have been studied.

Since an organic EL device using a polymer material can be prepared without using a vacuum unlike those requiring the vacuum vapor deposition, it is expected that thin films having an excellent quality can be easily formed. This provides a great advantage in the production process, and the advantageous preparation of a device having a great area is expected. However, there is a problem in that elution with a solvent takes place during the coating for forming the hole injecting layer, the hole transporting layer and the light emitting layer of the laminate structure. PEDOT/PSS having the structure shown below is excellent as the hole transporting layer since PEDOT/PSS is soluble in water and insoluble in organic solvents. However, the life of light emission is not always sufficient, and the improvement has been desired. As the factors adversely affecting the life of light emission, contamination with water and adverse effects of oxygen atom and sulfur atom formed by decomposition are suspected. 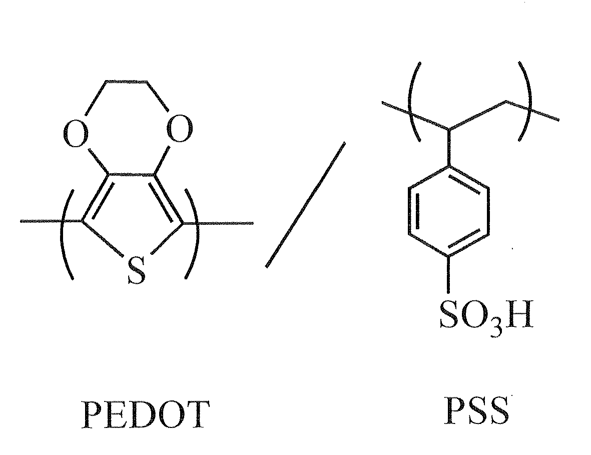
[Patent Reference 1] Japanese Patent Application Laid-Open No. Heisei 9(1997)-301934
[Patent Reference 2] International Patent Application Laid-Open No. WO98/30071
[Patent Reference 3] Japanese Patent Application Laid-Open No. 2000-309566
[Patent Reference 4] Japanese Patent Application Laid-Open No. Heisei 4(1992)-308688

### DISCLOSURE OF THE INVENTION

### PROBLEMS TO BE SOLVED BY THE INVENTION

The present invention has been made to overcome the above problems and has an object of providing an organic EL device exhibiting an excellent balance in physical properties such as a small ionization potential, a great band gap energy, a great efficiency of injection and a great mobility and excellent heat resistance and efficiency of light emission and a novel amine-based compound enabling to obtain the device.

### MEANS FOR SOLVING THE PROBLEM

As the result of intensive studies by the present inventors to achieve the above object, it was found that the above object could be achieved by using an amine-based compound represented by the following general formula (1) or (2) as the material for the organic EL device. The present invention has been completed based on the knowledge.

The present invention provides an amine-based compound represented by following general formula (1) or (2): 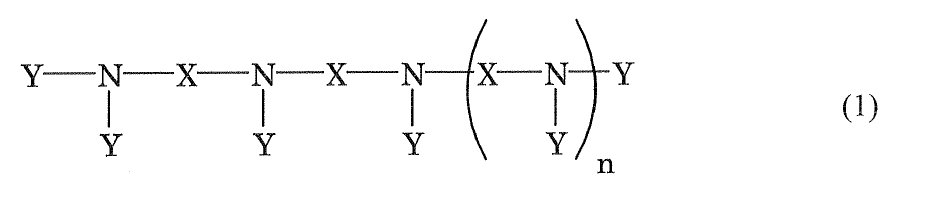 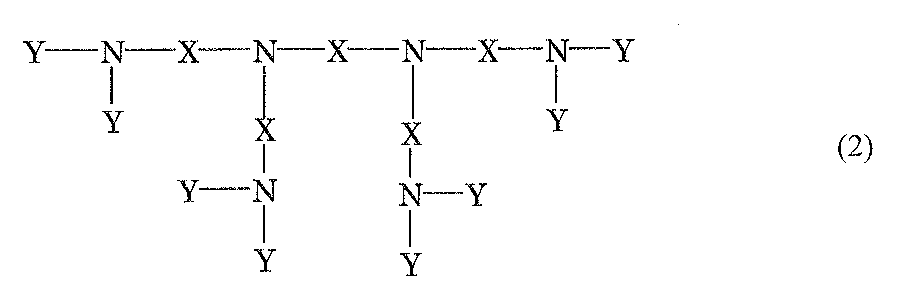 wherein X represents a substituted or unsubstituted arylene group having 4 to 50 carbon atoms, a heterocyclic crosslinking group or a group represented by following general formula (3) or (4), a plurality of X may represent a same group or different groups, and at least one of the plurality of X in general formulae (1) and (2) represents a group represented by following general formula (3) or (4);
Y represents a substituted or unsubstituted aryl group having 4 to 50 carbon atoms or a substituted or unsubstituted heterocyclic group having 5 to 50 carbon atoms, and a plurality of Y may represent a same group or different groups;
n represents an integer of 0 to 3; and N represents nitrogen atom; general formulae (3) and (4) being: 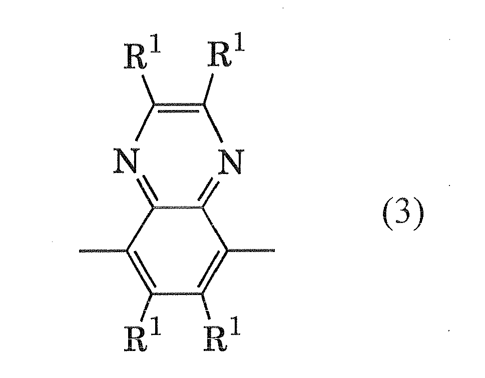 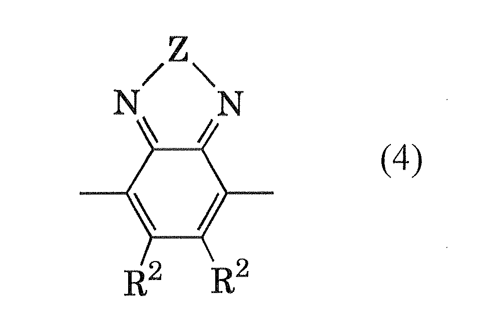 wherein R¹ and R² each independently represent hydrogen atom, a halogen atom, a substituted or unsubstituted alkyl group having 1 to 50 carbon atoms, a substituted or unsubstituted alkoxyl group having 1 to 50 carbon atoms, a substituted or unsubstituted aryloxyl group having 4 to 50 carbon atoms, a substituted or unsubstituted thioalkoxyl group having 1 to 50 carbon atoms, a substituted or unsubstituted thioaryloxyl group having 4 to 50 carbon atoms, a substituted or unsubstituted amino group, a substituted or unsubstituted aryl group having 4 to 50 carbon atoms, a substituted or unsubstituted alkylcarbonyl group having 1 to 50 carbon atoms or a substituted or unsubstituted arylcarbonyl group having 4 to 50 carbon atoms; atoms and groups represented by R¹ and R² may be same with or different from each other; and adjacent groups represented by R¹ may be bonded to each other to form a cyclic structure, and adjacent groups represented by R² may be bonded to each other to form a cyclic structure; and

Z represents sulfur atom, oxygen atom or -NR³-, R³ representing hydrogen atom, a substituted or unsubstituted aryl group having 4 to 50 carbon atoms, a substituted or unsubstituted heterocyclic group having 5 to 50 carbon atoms or a substituted or unsubstituted alkyl group having 1 to 50 carbon atoms.

The present invention also provides an organic EL device which comprises a cathode, an anode and an organic thin film layer which comprises at least one layer comprising at least a light emitting layer and is disposed between the cathode and the anode, wherein at least one layer in the organic thin film layer comprises the amine-based compound described above singly or as a component of a mixture.

### THE EFFECT OF THE INVENTION

The amine-based compound of the present invention is useful as the hole injecting material and the hole transporting material for organic EL devices and photo-sensitive materials for electronic photography. The organic EL device using the amine-based compound of the present invention exhibits an excellent balance in physical properties such as a small ionization potential, a great band gap energy, a great efficiency of injection and a great mobility and excellent heat resistance and efficiency of light emission.

### THE MOST PREFERRED EMBODIMENT TO CARRY OUT THE INVENTION

The amine-based compound of the present invention is represented by the following general formula (1) or (2):

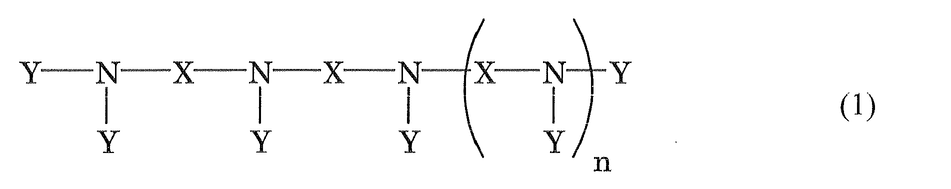

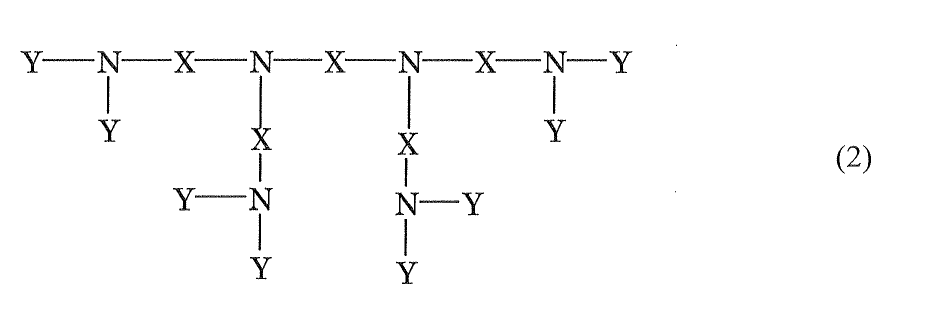

In general formulae (1) and (2), N represents nitrogen atom.

In general formula (1), n represents an integer of 0 to 3 and preferably 1.

In general formulae (1) and (2), Y represents a substituted or unsubstituted aryl group having 4 to 50 carbon atoms or a substituted or unsubstituted heterocyclic group having 5 to 50 carbon atoms, and a plurality of Y may represent the same group or different groups.

Examples of the aryl group represented by Y include phenyl group, substituted phenyl groups such as various types of tolyl groups and various types of xylyl groups, 1-naphthyl group, substituted 1-naphthyl groups such as various types of methyl-substituted 1-naphthyl groups and various types of dimethyl-substituted 1-naphthyl groups, 2-naphthyl group, substituted 2-naphthyl groups such as various types of methyl-substituted 2-naphthyl groups and various types of dimethyl-substituted 2-naphthyl groups, (substituted) 1-anthryl groups, (substituted) 2-anthryl groups, (substituted) 9-anthryl groups, (substituted) 1-phenanthryl groups, (substituted) 2-phenanthryl groups, (substituted) 3-phenanthryl groups, (substituted) 4-phenanthryl groups, (substituted) 9-phenanthryl groups, (substituted) 1-naphthacenyl groups, (substituted) 2-naphthacenyl groups, (substituted) 9-naphthacenyl groups, (substituted) 1-pyrenyl groups, (substituted) 2-pyrenyl groups and (substituted) 4-pyrenyl groups. Among these groups, (substituted) phenyl groups and (substituted) naphthyl groups are preferable.

The substituent to the above groups is not limited to hydrocarbon groups but may be groups having a heteroatom such as alkoxyl groups, carboxyl group, carboxyester groups, aryloxyl groups, dialkylamino groups, diarylamino groups and thioalkoxyl groups. The atoms constituting the nucleus are not limited to carbon atoms and may contain a heteroatom such as oxygen atom (furyl group and the like), nitrogen atom (pyridyl group, pyrrolyl group and the like), boron atom (boraphenyl group and the like), silicon atom (silaphenyl group and the like) and sulfur atom (thiofuryl group and the like).

Examples of the heterocyclic group represented by Y include pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triatinyl group, carbazolyl group, furanyl group, thiophenyl group, oxazolyl group, oxadiazolyl group, benzoxazolyl group, thiazolyl group, thiadiazolyl group, benzothiazolyl group, triazolyl group, imidazolyl group, benzimidazolyl group and purinyl group. The above groups may have substituents. Examples of the substituent include substituents described above as the examples of the substituent to the aryl group.

In general formulae (1) and (2), X represents a substituted or unsubstituted arylene group having 4 to 50 carbon atoms, a hetero cyclic crosslinking group or a group represented by the following general formula (3) or (4), a plurality of X may represent the same group or different groups, and at least one of the plurality of X in general formulae (1) and (2) represents a group represented by the following general formula (3) or (4).

Examples of the arylene group represented by X include phenylene group, substituted phenylene groups such as various types of tolylene group and various types of xylylene group, various types of naphthylene groups, substituted naphthylene groups such as various types of methyl-substituted naphthylene groups and various types of dimethyl-substituted naphthylene groups, various types of (substituted) anthrylene groups, various types of (substituted) phenanthrylene groups, various types (substituted) 1-naphthacenylene groups and various types of (substituted) pyrenylene groups. Among these groups, (substituted) phenylene groups and (substituted) naphthylene groups are preferable.

The positions of the crosslinking are not particularly limited. It is preferable that the crosslinking is formed at the 1- and 4-positions.

Examples of the heterocyclic crosslinking group represented by X include (substituted) pyridinylene groups, (substituted) pyrrolylene groups, (substituted) thiophenylene groups and (substituted) furanylene groups. Among these groups, (substituted) pyridinylene groups, (substituted) pyrrolylene groups and (substituted) thiophenylene groups are preferable, and (substituted) pyridinylene groups crosslinked at the 2,5-positions, (substituted) pyrrolylene groups crosslinked at the 2,5-posations and (substituted) thiophenylene groups crosslinked at the 2,5-positions are more preferable.

The substituent to the above groups is not limited to hydrocarbon groups but may be groups having a heteroatom such as alkoxylene groups, carboxyester groups, aryloxylene groups, dialkylamino groups, and thioalkoxylene groups. The atoms constituting the nucleus are not limited to carbon atoms and may contain a heteroatom such as oxygen atom (furylene group and the like), nitrogen atom (pyridylene group, pyrrolylene group and the like), boron atom (boraphenylene group and the like), silicon atom (silaphenylene group and the like) and sulfur atom (thiofurylene group and the like).

General formulae (3) and (4) are shown in the following:

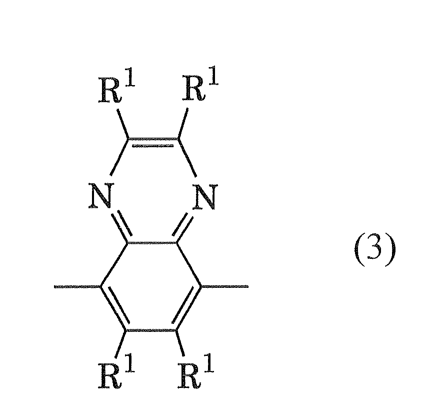

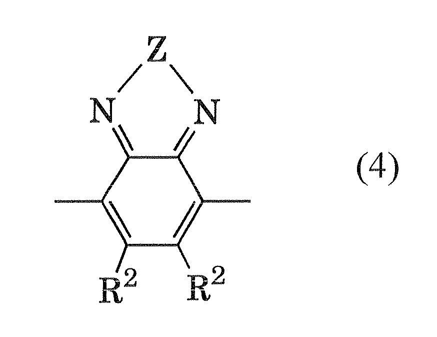

In general formulae (3) and (4), R¹ and R² each independently represent hydrogen atom, a halogen atom, a substituted or unsubstituted alkyl group having 1 to 50 carbon atoms, a substituted or unsubstituted alkoxyl group having 1 to 50 carbon atoms, a substituted or unsubstituted aryloxyl group having 4 to 50 carbon atoms, a substituted or unsubstituted thioalkoxyl group having 1 to 50 carbon atoms, a substituted or unsubstituted thioaryloxyl group having 4 to 50 carbon atoms, a substituted or unsubstituted amino group, a substituted or unsubstituted aryl group having 4 to 50 carbon atoms, a substituted or unsubstituted alkylcarbonyl group having 1 to 50 carbon atoms or a substituted or unsubstituted arylcarbonyl group having 4 to 50 carbon atoms. Atoms or groups represented by R¹ and R² may be the same with or different from each other. Adjacent groups represented by R¹ may be bonded to each other to form a cyclic structure, and adjacent groups represented by R² may be bonded to each other to form a cyclic structure.

Examples of the halogen atom represented by R¹ and R² include fluorine atom, chlorine atom, bromine atom and iodine atom.

Examples of the alkyl group represented by R¹ and R² include methyl group, ethyl group, propyl group, isopropyl group, n-butyl group, s-butyl group, isobutyl group, t-butyl group, n-pentyl group, n-hexyl group, n-heptyl group and n-octyl group.

Examples of the aryl group represented by R¹ and R² include phenyl group, substituted phenyl groups such as various types of tolyl groups and various types of xylyl groups, 1-naphthyl group, substituted 1-naphthyl groups such as various types of methyl-substituted 1-naphthyl groups and various types of dimethyl-substituted 1-naphthyl groups, 2-naphthyl group, substituted 2-naphthyl groups such as various types of methyl-substituted 2-naphthyl groups and various types of dimethyl-substituted 2-naphthyl groups, (substituted) 1-anthryl groups, (substituted) 2-anthryl groups, (substituted) 9-anthryl groups, (substituted) 1-phenanthryl groups, (substituted) 2-phenanthryl groups, (substituted) 3-phenanthryl groups, (substituted) 4-phenanthryl groups, (substituted) 9-phenanthryl groups, (substituted) 1-naphthacenyl groups, (substituted) 2-naphthacenyl groups, (substituted) 9-naphthacenyl groups, (substituted) 1-pyrenyl groups, (substituted) 2-pyrenyl groups and (substituted) 4-pyrenyl groups.

The alkoxyl group represented by R¹ and R² is represented by RO-. Examples of the group represented by R include the groups described above as the examples of the alkyl group.

The aryloxyl group represented by R¹ and R² is represented by R'O-. Examples of the group represented by R' include the groups described above as the examples of the aryl group.

Examples of the thioalkoxyl group represented by R¹ and R² include the groups similar to those described above as the examples of the alkoxyl group.

Examples of the thioaryloxyl group represented by R¹ and R² include the groups similar to those described above as the examples of the aryloxyl group.

Examples of the amino group represented by R¹ and R² include diphenylamino group, ditolylamino group, dinaphthylamino group, naphthylphenylamino group, dimethylamino group, diethylamino group and dihexylamino group.

The alkylcarbonyl group represented by R¹ and R² is represented by RCO-. Examples of the group represented by R include the groups described above as the examples of the alkyl group.

The arylcarbonyl group represented by R¹ and R² is represented by R'CO-. Examples of the group represented by R' include the groups described above as the examples of the aryl group.

When adjacent groups represented by a plurality of R¹ or R² are bonded to each other to form a cyclic structure, examples of the group include (substituted) 1,1-biphenylene groups, (substituted) 1,5-naphthylene groups, (substituted) 1,4-butylene groups and (substituted) 1,4-butadienylene groups.

In general formula (4), Z represents sulfur atom, oxygen atom or -NR³-, wherein R³ represents hydrogen atom, a substituted or unsubstituted aryl group having 4 to 50 carbon atoms, a substituted or unsubstituted heterocyclic group having 5 to 50 carbon atoms or a substituted or unsubstituted alkyl group having 1 to 50 carbon atoms.

Examples of the aryl group, the heterocyclic group and the alkyl group represented by R³ include the corresponding groups described above as the examples of the aryl group, the heterocyclic group and the alkyl group, respectively.

Specific examples of the amine-based compound represented by general formula (1) or (2), which is the amine-based compound of the present invention, are shown in the following. However, the amine-based compound is not limited to the compounds shown as the examples.

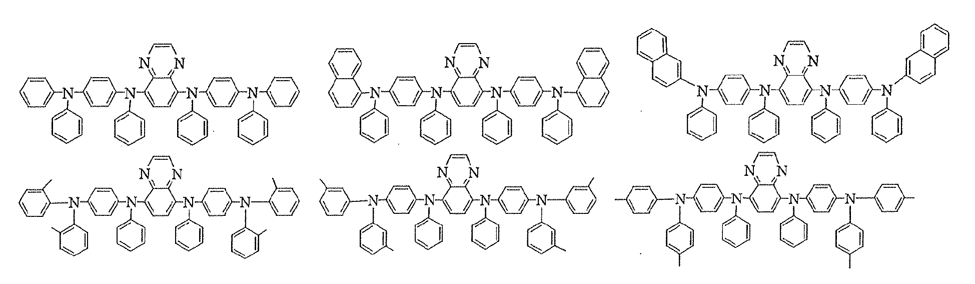

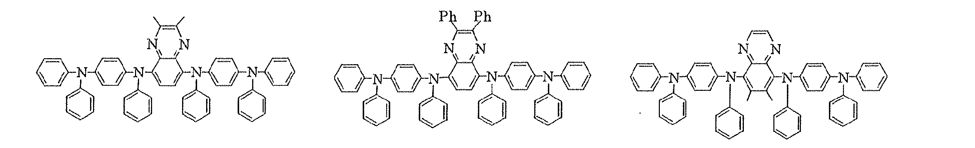

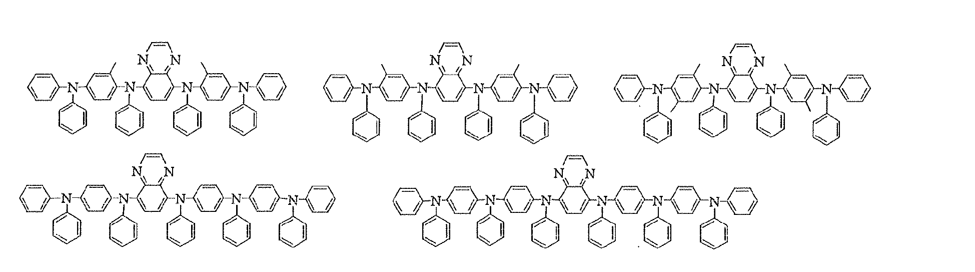

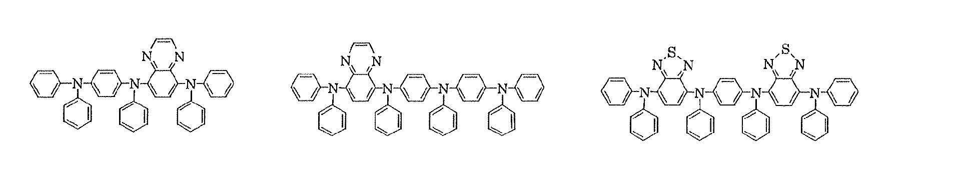

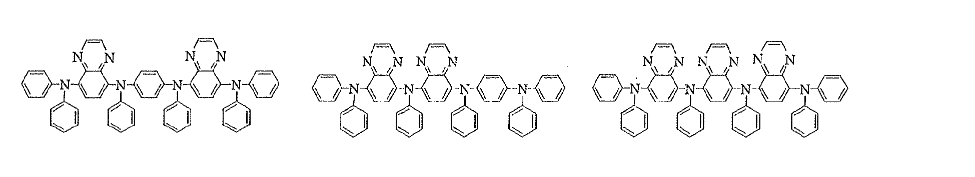

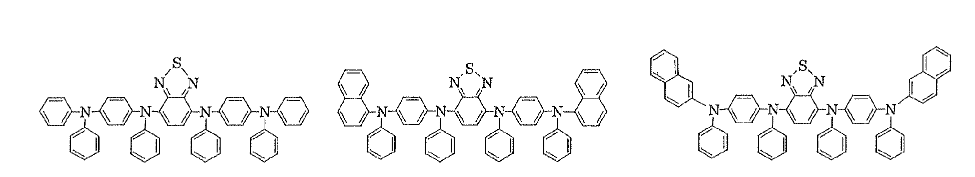

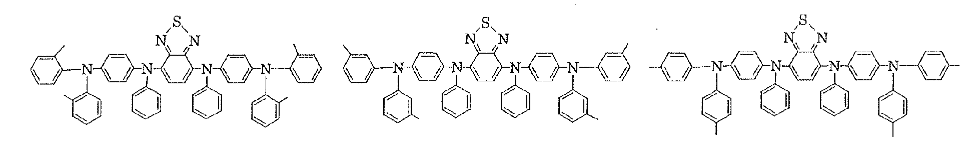

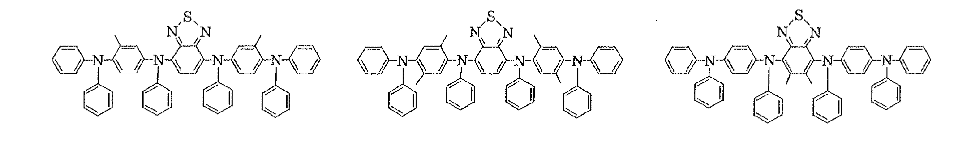

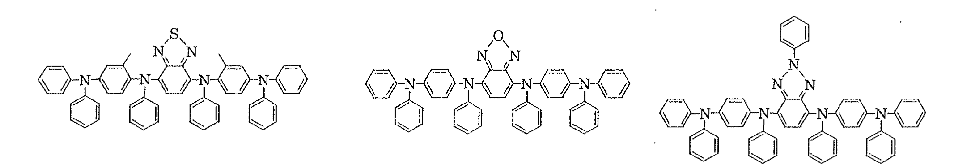

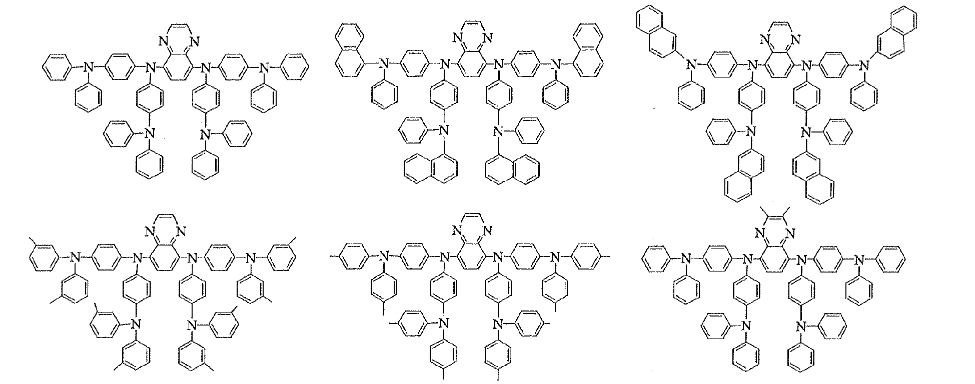

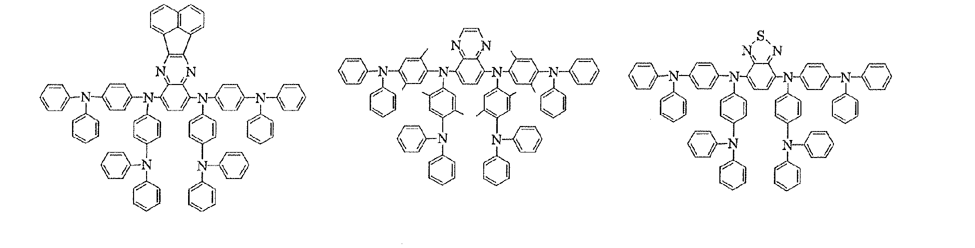

The process for producing the amine-based compound of the present invention will be described in the following,

The amine-based compound of the present invention can be produced in accordance with a conventional process (the reference is, for example, Yuki Gosei Kagaku Kyokai Shi, Volume 59, Number 6, 2001, Page 607). Processes for synthesis [1] to [4] for typical groups of the compounds are shown in the following. However, the process for the synthesis is not limited to the processes shown as the examples.

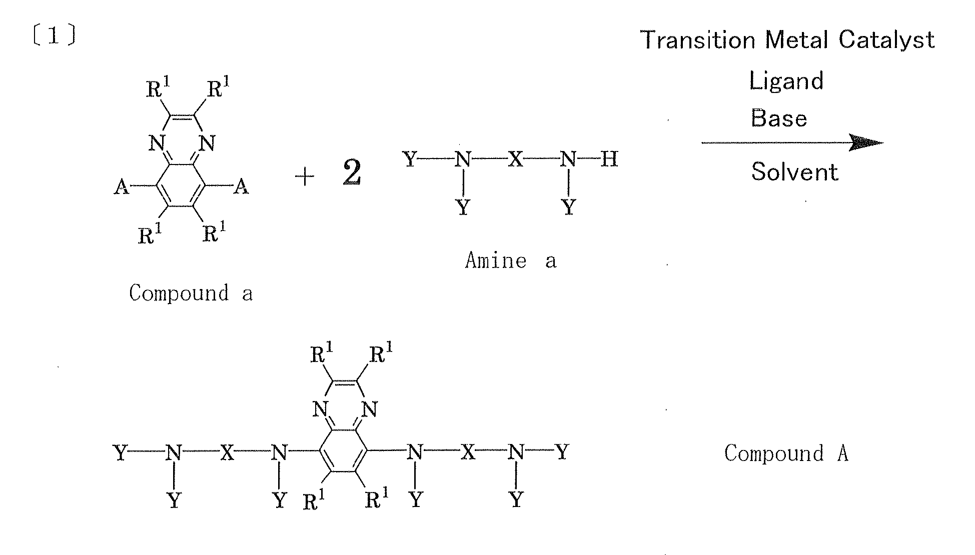

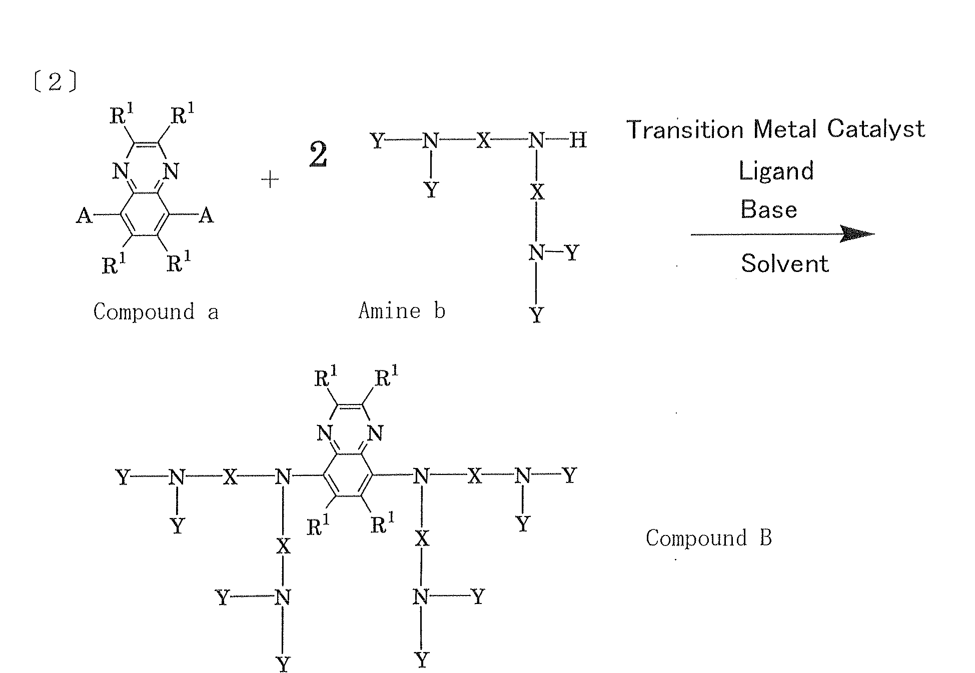

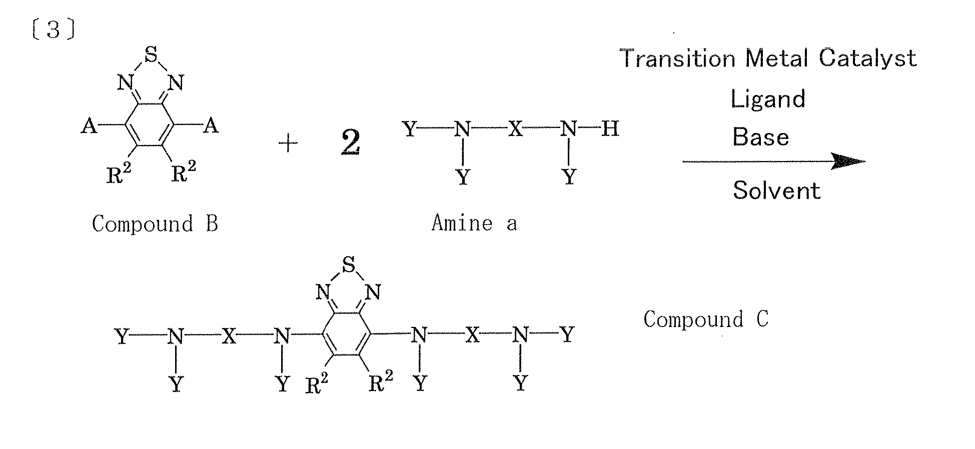

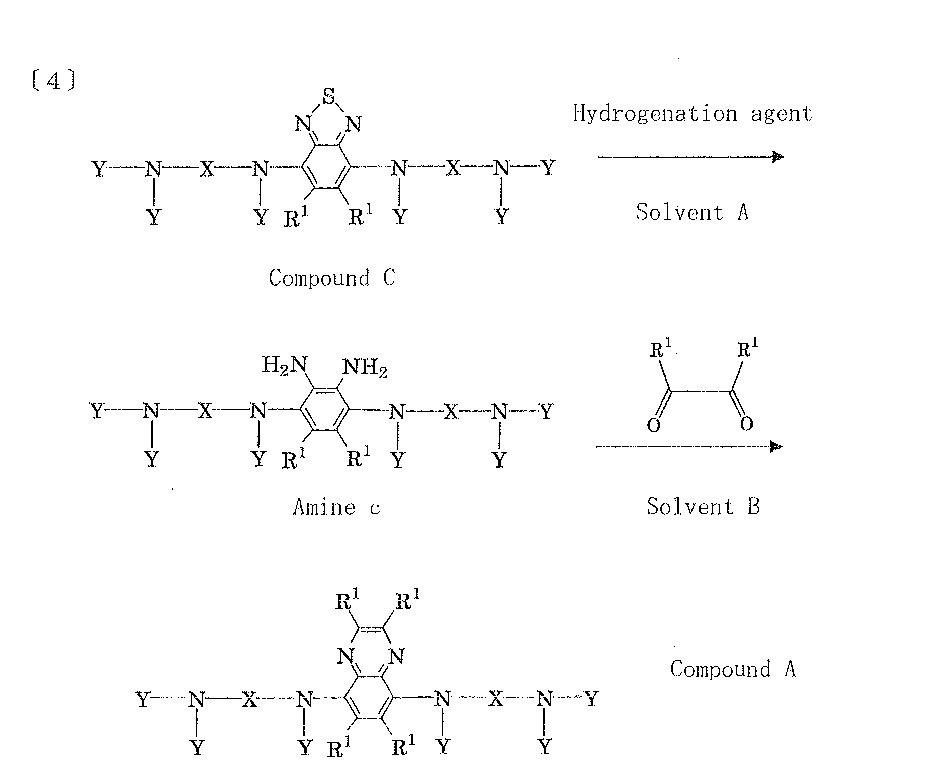

Compounds and reagents used in processes of synthesis [1] to [4] will be described in the following.

In Compounds a to c, amines a to c and Compounds A to C, N represents nitrogen atom, and X, Y, R¹ and R² are as defined above. A represents a halogen atom, preferably iodine atom, bromine atom or chlorine atom and more preferably bromine atom.

The transition metal compound is a compound having a transition atom selected from metals of Group 8, Group 9 and Group 10 of the Periodic Table, preferably a compound having a transition metal selected from metals of Group 10 and more preferably a compound having palladium. Specific examples include PdCl₂, Pd(OAc)₂ (Ac: acetyl group), Pd₂(dba)₃ (dba: dibenzylyleneacetone), BINAP (2,2'-bis(diphenyl-phosphino)-1,1'-binaphthyl) and DPPF (1,1'-bis(diphenylphosphino)-ferrocene).

The ligand is a compound having an element of Group 15 or Group 16, preferably a compound having an element of Group 15 and more preferably a compound having phosphorus. Specific examples of the ligand include PAr₃ (Ar: an aryl group) such as P(o-Tol)₃ (Tol: tolyl group) and PPh₃ (Ph: phenyl group) and PR₃ (R: an alkyl group) such as PCy₃ (Cy: cyclohexyl group) and PtBu₃.

The base is a compound comprising an alkali metal and a conjugate base or an alkaline earth metal and a conjugate base, and any base can be used as long as hydrogen atom in the amine used for the reaction can be dissociated as proton. A conjugate base of a basic compound having an acid dissociation constant (a pKa value) of 18 or greater (at 25°C in water) is preferable.

As the alkali metal, lithium and sodium are preferable and, as the alkaline earth metal, magnesium is preferable.

Examples of the conjugate base of a basic compound having an acid dissociation constant (a pKa value) of 18 or greater (at 25°C in water) include -N(SiR₃)₂, -NR₂ and -OR (R: an alkyl group). Among these bases, -N(SiMe₃)₂ (Me: methyl group), -N(isopropyl)₂ and -OtBu are preferable.

The solvent is not particularly limited as long as the solvent does not react with the compound of the transition metal, the ligand or the base. Aromatic solvents such as xylenes and toluene are preferable.

The temperature of the reaction is not particularly limited. The temperature is, in general, in the range of the room temperature to 200°C or to the boiling point of the solvent and preferably in the range of the room temperature to 120°C.

The time of the reaction is not particularly limited. The time is, in general, 1 to 150 hours and preferably 3 to 100 hours.

The relative amounts of the reactants are not particularly limited. The ratio of the amounts by mole of Compounds a, b or c : amine a, b or c : the compound of the transition metal : the ligand : the base is, in general, 100 : 180~300 : 0.1~10 : 0.1~40 : 150~300 and preferably 100 : 190~220 : 0.5~5 : 1~10 : 180~250.

The concentration of the reaction fluid is not particularly limited. The concentration of the reaction fluid expressed as the concentration of Compound a, b or c is, in general, 0.01 to 2 moles/liter and preferably 0.1 to 0.2 moles/liter.

The amine-based compound of the present invention is suitable for the hole injecting material and the hole transporting material and can be used in a wide area of applications as the material for the solar batteries, photo-sensitive materials for electronic photography and organic EL devices. In particular, the amine-based compound is suitable for the hole injecting material and the hole transporting material of organic EL devices.

The organic electroluminescence device of the present invention comprises a cathode, an anode and an organic thin film layer which comprises at least one layer comprising at least a light emitting layer and is disposed between the cathode and the anode, wherein at least one layer in the organic thin film layer comprises the amine-based compound of the present invention singly or as a component of a mixture.

It is preferable that, in the organic EL device of the present invention, the organic thin film layer comprises a hole injecting layer and/or a hole transporting layer, and the hole injecting layer and/or the hole transporting layer comprises the amine-based compound of the present invention singly or as a component of a mixture.

The construction of the organic EL device of the present invention will be described in the following.

Typical examples of the construction of the organic EL device include:
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) An anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode.

Among the above constructions, construction (8) is preferable. However, the construction of the organic EL device is not limited to those shown above as the examples.

It is preferable that the light emitting zone or the hole transporting zone comprises the amine-based compound of the present invention among the constituting elements of the device although any of the organic layers may comprise the amine-based compound. It is more preferable that the hole injecting zone comprises the amine-based compound.

In the organic EL device of the present invention, the light emitting layer may have a single layer or a plurality of layers, and it is preferable that the light emitting layer comprises the amine-based compound of the present invention an amount in the range of 30 to 100% by mole.

The organic EL device is, in general, prepared on a substrate transmitting light. The substrate transmitting light is the substrate supporting the organic EL device. It is preferable that the substrate transmitting light has a transmittance of light of 50% or greater in the visible region of 400 to 700 nm. It is also preferable that a flat and smooth substrate is used.

As the substrate transmitting light, for example, glass plates and synthetic resin plates are advantageously used. Examples of the glass plate include plates made of soda lime glass, glass containing barium and strontium, lead glass, aluminosilicate glass, borosilicate glass, barium borosilicate glass and quartz. Examples of the synthetic resin plate include plates made of polycarbonate resins, acrylic resins, polyethylene terephthalate resins, polyether sulfide resins and polysulfone resins.

The anode has the function of injecting holes into the hole transporting layer or the light emitting layer. It is effective that the anode has a work function of 4.5 eV or greater. Examples of the material for the anode used in the present invention include indium tin oxide alloys (ITO), indium-zinc alloys (IZO), tin oxide (NESA), gold, silver, platinum and copper. For the cathode, materials having a small work function are preferable for the purpose of injecting electrons into the electron transporting layer or the light emitting layer.

The anode can be prepared by forming a thin film of the above electrode material in accordance with a process such as the vapor deposition process and the sputtering process.

When the light emitted from the light emitting layer is obtained through the anode, it is preferable that the anode has a transmittance of the emitted light greater than 10%. It is also preferable that the sheet resistivity of the anode is several hundred Ω/ □ or smaller. The thickness of the anode is, in general, selected in the range of 10 nm to 1 µm and preferably in the range of 10 to 200 nm although the thickness may be different depending on the used material.

The light emitting layer in the organic EL device of the present invention has the following functions:
(i) The injecting function: the function of injecting holes from the anode or the hole injecting layer and injecting electrons from the cathode or the electron injecting layer when an electric field is applied;
(ii) The transporting function: the function of transporting injected charges (electrons and holes) by the force of the electric field; and
(iii) The light emitting function: the function of providing the field for recombination of electrons and holes and leading the recombination to the emission of light. The easiness of injection of holes and the easiness of injection of electrons may be different. The ability of transportation expressed by the mobility may be different between holes and electrons. It is preferable that one of the charges is transferred.

As the process for forming the light emitting layer, a conventional process such as the vapor deposition process, the spin coating process and the LB process can be used. It is preferable that the light emitting layer is a molecular deposit film.

The molecular deposit film is a thin film formed by deposition of a material compound in the gas phase or a thin film formed by solidification of a material compound in a solution or in the liquid phase. In general, the molecular deposit film can be distinguished from the thin film formed in accordance with the LB process (the molecular accumulation film) based on the differences in aggregation structures and higher order structures and the functional differences caused by these structural differences.

As disclosed in Japanese Patent Application Laid-Open No. Showa 57(1982)-51781, the light emitting layer can also be formed by dissolving a binder such as a resin and the material compounds into a solvent to prepare a solution, followed by forming a thin film from the prepared solution in accordance with the spin coating process or the like.

In the present invention, where desired, the light emitting layer may comprise conventional light emitting materials other than the light emitting material comprising the amine-based compound of the present invention, or a light emitting layer comprising other conventional light emitting material may be laminated to the light emitting layer comprising the light emitting material of the present invention as long as the object of the present invention is not adversely affected.

In organic EL device of the present invention, it is preferable that the light emitting layer is a layer emitting bluish light. It is preferable that light emitting layer has a maximum wavelength of the light emission of 450 to 500 nm and comprises a host material and a bluish dopant.

As the host material in the light emitting layer, styryl derivatives, arylene derivatives and aromatic amines (amine derivatives) are preferable.

It is preferable that the styryl derivative is at least one compound selected from distyryl derivatives, tristyryl derivatives, tetrastyryl derivatives and styrylamine derivatives.

It is preferable that the arylene derivative is an anthracene derivative and more preferably a compound having a skeleton structure of an arylanthracene.

It is preferable that the aromatic amine is a compound having 2 to 4 nitrogen atoms substituted with an aromatic group and more preferably a compound having 2 to 4 nitrogen atoms substituted with an aromatic group and at least one alkenyl group.

Examples of the styryl derivative and the anthracene derivative include compounds represented by the following general formulae [1] to [6]. Examples of the aromatic amine include compounds represented by the following general formulae [7] and [8].

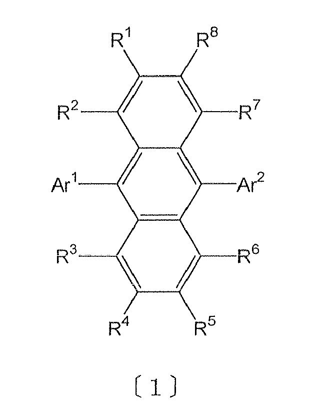

In the above general formula [1], R¹ to R⁸ each independently represent hydrogen atom, a halogen atom, cyano group, nitro group, a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted condensed polycyclic group having 10 to 30 carbon atoms or a substituted or unsubstituted heterocyclic group having 5 to 30 carbon atoms. Ar¹ and Ar² each independently represent a substituted or unsubstituted aryl group having 6 to 30 carbon atoms or a substituted or unsubstituted alkenyl group. The substituent is a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted condensed polycyclic group having 10 to 30 carbon atoms or a substituted or unsubstituted heterocyclic group having 5 to 30 carbon atoms.

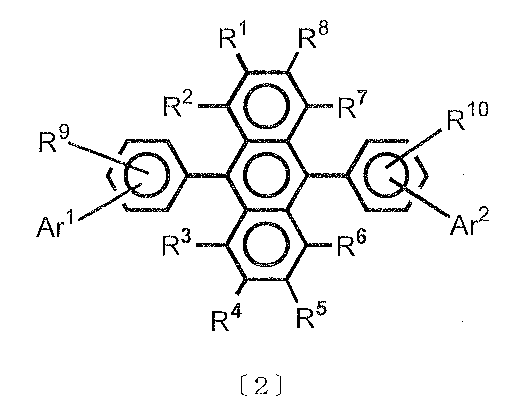

In the above general formula [2], R¹ to R¹⁰ each independently represent hydrogen atom, a halogen atom, cyano group, nitro group, a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted condensed polycyclic group having 10 to 30 carbon atoms or a substituted or unsubstituted heterocyclic group having 5 to 30 carbon atoms. Ar¹ and Ar² each independently represent a substituted or unsubstituted aryl group having 6 to 30 carbon atoms or a substituted or unsubstituted alkenyl group. The substituent is a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted condensed polycyclic group having 10 to 30 carbon atoms or a substituted or unsubstituted heterocyclic group having 5 to 30 carbon atoms.

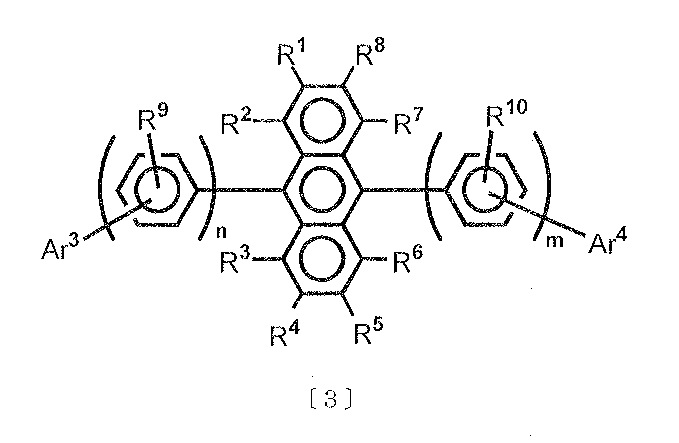

In the above general formula [3], R¹ to R¹⁰ each independently represent hydrogen atom, a halogen atom, cyano group, nitro group, a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted condensed polycyclic group having 10 to 30 carbon atoms or a substituted or unsubstituted heterocyclic group having 5 to 30 carbon atoms. Ar³ and Ar⁴ each independently represent a substituted or unsubstituted aryl group having 6 to 30 carbon atoms or a substituted or unsubstituted alkenyl group. The substituent is a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted condensed polycyclic group having 10 to 30 carbon atoms, a substituted or unsubstituted heterocyclic group having 5 to 30 carbon atoms or a substituted or unsubstituted alkenyl group having 4 to 40 carbon atoms. n represents an integer of 1 to 3, m represents an integer of 1 to 3, and n+m≧2.

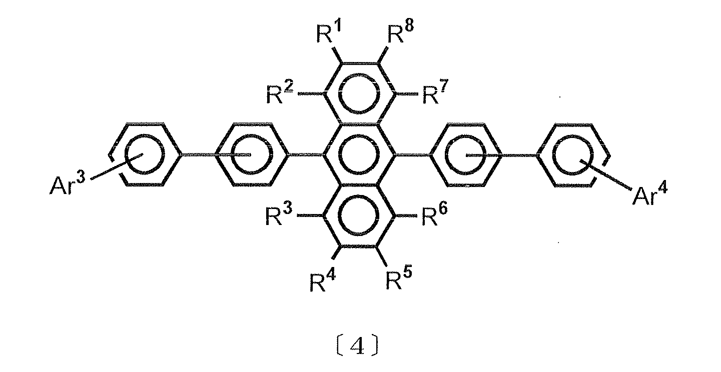

In the above general formula [4], R¹ to R⁸ each independently represent hydrogen atom, a halogen atom, cyano group, nitro group, a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted condensed polycyclic group having 10 to 30 carbon atoms or a substituted or unsubstituted heterocyclic group having 5 to 30 carbon atoms. Ar³ and Ar⁴ each independently represent a substituted or unsubstituted aryl group having 6 to 30 carbon atoms or a substituted or unsubstituted alkenyl group. The substituent is a substituted or unsubstituted alkyl group having 1 to 20 carbon atoms, a substituted or unsubstituted alkoxyl group having 1 to 20 carbon atoms, a substituted or unsubstituted aryloxyl group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted condensed polycyclic group having 10 to 30 carbon atoms, a substituted or unsubstituted heterocyclic group having 5 to 30 carbon atoms or a substituted or unsubstituted alkenyl group having 4 to 40 carbon atoms.

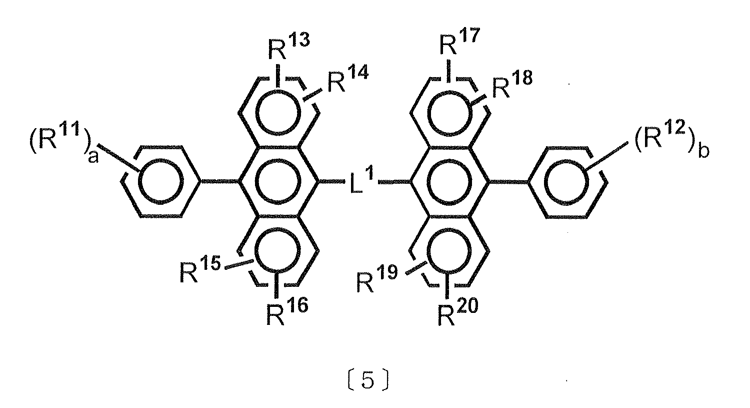

In the above general formula [5], R¹¹ to R²⁰ each independently represent hydrogen atom, an alkenyl group, an alkyl group, a cycloalkyl group, an aryl group, an alkoxyl group, an aryloxyl group, an alkylamino group, an arylamino group or a heterocyclic group which may be substituted. a and b each represent an integer of 1 to 5. When a or b represent an integer of 2 or greater, atoms and groups represented by the plurality of R¹¹ or R¹², respectively, may be the same with or different from each other, and the groups represented by R¹¹ or R¹², respectively, may be bonded to each other to form a ring. Groups represented by the pair of R¹³ and R¹⁴, the pair of R¹⁵ and R¹⁶, the pair of R¹⁷ and R¹⁸ or the pair of R¹⁹ and R²⁰ may be bonded to each other to form a ring. L¹ represents the single bond, -O-, -S-, -N(R), R representing an alkyl group or an aryl group which may be substituted, or an arylene group.

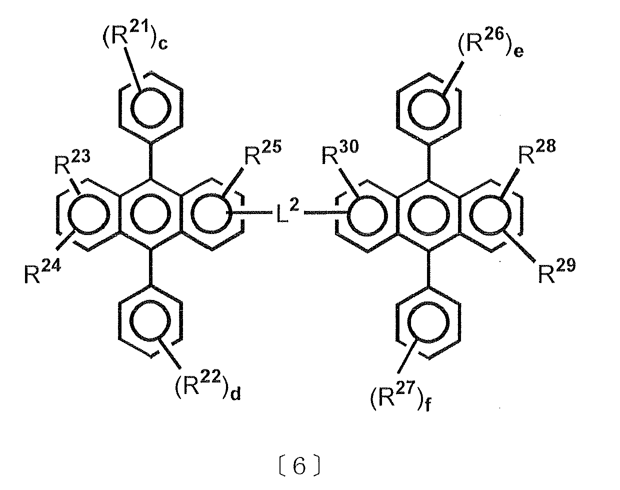

In the above general formula [6], R²¹ to R³⁰ each independently represent hydrogen atom, an alkenyl group, an alkyl group, a cycloalkyl group, an aryl group, an alkoxyl group, an aryloxyl group, an alkylamino group, an arylamino group or a heterocyclic group which may be substituted. c, d, e and f each represent an integer of 1 to 5. When c, d, e or f represent an integer of 2 or greater, atoms and groups represented by the plurality of R²¹, R²², R²⁶ or R²⁷, respectively, may be the same with or different from each other, and the groups represented by R²¹, R²², R²⁶ or R²⁷, respectively, may be bonded to each other to form a ring. Groups represented by the pair of R²³ and R²⁴ or the pair of R²⁸ and R²⁹ may be bonded to each other to form a ring. L² represents the single bond, -O-, -S-, -N(R)-, R representing an alkyl group or an aryl group which may be substituted, or an arylene group.

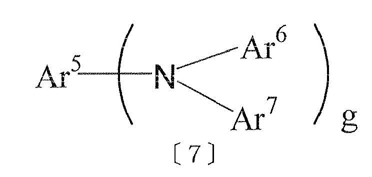

In the above general formula [7], Ar⁵, Ar⁶ and Ar⁷ each independently represent a substituted or unsubstituted monovalent aromatic group having 6 to 40 carbon atoms or styryl group, and g represent an integer of 1 to 4.

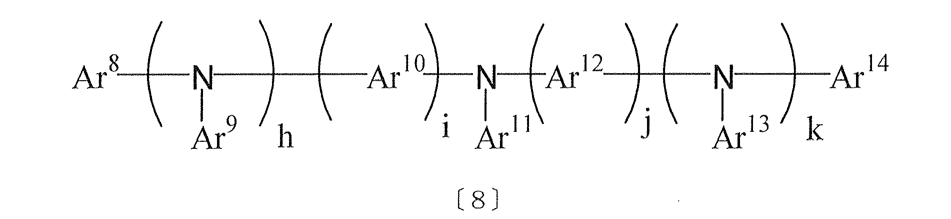

In the above general formula [8], Ar⁸, Ar⁹, Ar¹¹, Ar¹³ and Ar¹⁴ each independently represent a substituted or unsubstituted monovalent aromatic group having 6 to 40 carbon atoms or styryl group, Ar¹⁰ and Ar¹² each independently represent a substituted or unsubstituted divalent aromatic group having 6 to 40 carbon atoms or styrylene group, h and k each represent an integer of 0 to 2, and i and j reach represent an integer of 0 to 3.

As the dopant described above in the light emitting layer, at least one compound selected from amine derivatives such as styrylamine, styryl compounds substituted with an amine and compounds having a condensed aromatic ring is preferable. Examples of the styrylamine and styryl compound substituted with an amine include compounds represented by the following general formulae [9] and [10]. Examples of the compound having a condensed aromatic ring include compounds represented by the following general formula [11].

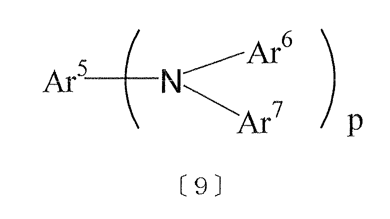

In the above general formula [9], Ar⁵, Ar⁶ and Ar⁷ each independently represent a substituted or unsubstituted aromatic group having 6 to 40 carbon atoms or styryl group, and p represents an integer of 1 to 3.

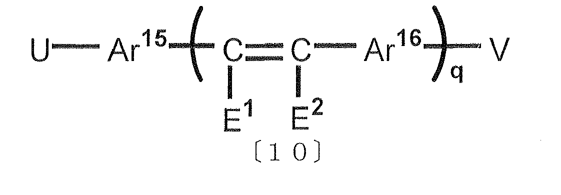

In the above general formula [10], Ar¹⁵ and Ar¹⁶ each independently represent an arylene group having 6 to 30 carbon atoms, E¹ and E² each independently represent an aryl group having 6 to 30 carbon atoms, an alkyl group, hydrogen atom or cyano group, q represents an integer of 1 to 3, and U and/or V represents a substituent having an amino group, the amino group being preferably an arylamino group.

(A)ᵣ-B [11]

In the above general formula [11], A represents an alkyl group or alkoxyl group having 1 to 16 carbon atoms, a substituted or unsubstituted aryl group having 6 to 30 carbon atoms, a substituted or unsubstituted alkylamino group having 6 to 30 carbon atoms or a substituted or unsubstituted arylamino group having 6 to 30 carbon atoms, B represents a condensed aromatic cyclic group having 10 to 40 carbon atoms, and r represents an integer of 1 to 4.

In the organic EL device of the present invention, a phosphorescent compound may be used in the light emitting layer. It is preferable that the host material comprising a phosphorescent compound is a compound having carbazole ring.

The host material comprising the compound having carbazole ring which is suitable for emission of phosphorescent light is a compound having a function such that the phosphorescent compound emits light as the result of transfer of energy to the phosphorescent compound from the host compound in the excited state. The host compound is not particularly limited as long as the energy of the excimer can be transferred to the phosphorescent compound and can be suitably selected in accordance with the object. The host compound may have any desired heterocyclic ring in combination with carbazole ring.

Examples of the host compound include carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidene-based compounds, porphyrin-based compounds, anthraquinodimethane derivatives, anthrone derivatives, diphenylquinone derivatives, thiopyrane dioxide derivatives, carbodiimide derivatives, fluorenylidenemethane derivatives, distyrylpyrazine derivatives, anhydrides of heterocyclic tetracarboxylic acid such as acids derived from naphthalene and perylene, phthalocyanine derivatives, various metal complexes such as metal complexes of 8-quinolinol, metal phthalocyanines and metal complexes using benzoxazole or benzothiazole as the ligand, polysilane-based compounds, poly(N-vinylcarbazole) derivatives, electrically conductive macromolecular oligomers such as aniline-based copolymers, thiophene oligomers and polythiophene, and polymer compounds such as polythiophene derivatives, polyphenylene derivatives, polyphenylene-vinylene derivatives and polyfluorene derivatives. The host compound may be used singly or in combination of two or more.

Examples of the host compound include compounds shown in the following:

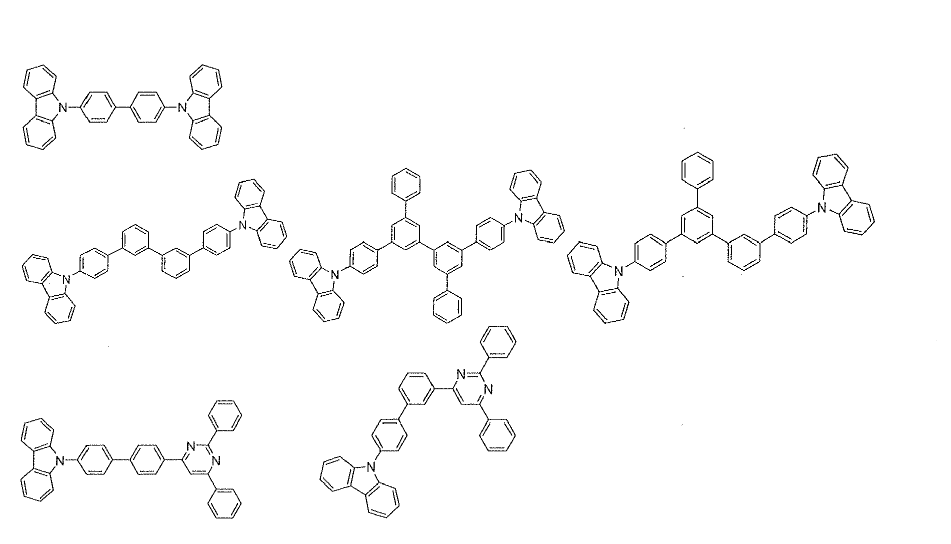

As the dopant comprising a phosphorescent compound, compounds which can emit light from the triplet excimers are preferable. The dopant compound is not particularly limited as long as light is emitted from the triplet excimers. Metal complexes having at least one metal selected from Ir, Ru, Pd, Pt, Os and Re are preferable. Porphyrin metal complexes and metal complexes in the form of an ortho-metal are more preferable. As the porphyrin metal complex, porphyrin platinum complexes are preferable. The phosphorescent compound may be used singly or in combination of two or more.

As the ligand for forming the metal complex in the form of an ortho-metal, various ligands can be used. Preferable examples of the ligand include 2-phenylpyridine derivatives, 7,8-benzoquinoline derivatives, 2-(2-thienyl)pyridine derivatives, 2-(1-naphthyl)pyridine derivatives and 2-phenylquinoline derivatives. These derivatives may have substituents, where necessary. As the bluish dopant, fluorides and derivatives having trifluoromethyl group introduced therein are preferable. The complexes may have ligands other than those described above such as acetylacetone and picric acid as the auxiliary ligand.

The content of the phosphorescent dopant in the light emitting layer is not particularly limited and can be suitably adjusted in accordance with the object. The content is, for example, 0.1 to 70% by mass and preferably 1 to 30% by mass. When the content of the phosphorescent dopant is smaller than 0.1% by mass, the light emission is weak, and the effect of the phosphorescent dopant is not sufficiently exhibited. When the content of the phosphorescent dopant exceeds 70% by mass, the phenomenon called concentration quenching becomes significant, and the properties of the device deteriorate.

The light emitting layer may further comprise a hole transporting material, an electron transporting material and a binder, where necessary.

The thickness of the light emitting layer is preferably 5 to 50 nm, more preferably 7 to 50 nm and most preferably 10 to 50 nm. When the thickness is 5 nm or greater, the formation of the light emitting layer and the adjustment of the chromaticity are facilitated. When the thickness is 50 nm or smaller, there is no possibility of an increase in the driving voltage.

The hole injecting and transporting layer is a layer which helps injection of holes into the light emitting layer and transports holes to the light emitting region. The layer exhibits a great mobility of holes and, in general, has an ionization energy as small as 5.5 eV or smaller. For the hole injecting and transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.

When the amine derivative having a hetero ring of the present invention is used for the hole transporting zone (the hole injecting and transporting layer), the compound of the present invention may be used singly or as a mixture with other material to form the hole injecting and transporting layer.

The material mixed with the aromatic amine derivative of the present invention and used for forming the hole injecting and transporting layer is not particularly limited as long as the material has the preferable properties described above, and a material can be selected as desired from materials which are conventionally used as the charge transporting material of holes in photoconductive materials and conventional materials which are used for the hole injecting layer in organic EL devices.

Examples of the material for the hole injecting layer described above include triazole derivatives (United States Patent No. 3,112,197), oxadiazole derivatives (United States Patent No. 3,189,447), imidazole derivatives (Japanese Patent Application Publication No. Showa 37(1962)-16096), polyarylalkane derivatives (United States Patent Nos. 3,615,402, 3,820,989 and 3,542,544; Japanese Patent Application Publication Nos. Showa 45(1970)-555 and Showa 51 (1976)-10983; and Japanese Patent Application Laid-Open Nos. Showa 51(1976)-93224, Showa 55(1980)-17105, Showa 56(1981)-4148, Showa 55(1980)-108667, Showa 55(1980)-156953 and Showa 56(1981)-36656); pyrazoline derivatives and pyrazolone derivatives (United States Patent Nos. 3,180,729 and 4,278,746; and Japanese Patent Application Laid-Open Nos. Showa 55(1980)-88064, Showa 55(1980)-88065, Showa 49(1974)-105537, Showa 55(1980)-51086, Showa 56(1981)-80051, Showa 56(1981)-88141, Showa 57(1982)-45545, Showa 54(1979)-112637 and Showa 55(1980)-74546); phenylenediamine derivatives (United Sates Patent No. 3,615,404; Japanese Patent Application Publication Nos. Showa 51(1976)-10105, Showa 46(1971)-3712 and Showa 47(1972)-25336; and Japanese Patent Application Laid-Open Nos. Showa 54(1979)-53435, Showa 54(1979)-110536 and Showa 54(1979)-119925); arylamine derivatives (United States Patent Nos. 3,567,450, 3,180,703, 3,240,597, 3,658,520, 4,232,103, 4,175,961 and 4,012,376; Japanese Patent Application Publication Nos. Showa 49(1974)-35702 and Showa 39(1964)-27577; Japanese Patent Application Laid-Open Nos. Showa 55(1980)-144250, Showa 56(1981)-119132 and Showa 56(1981)-22437; and West German Patent No. 1,110,518); chalcone derivatives substituted with amino group (United States Patent No. 3,526,501); oxazole derivatives (United States Patent No. 3,257,203); styrylanthracene derivatives (Japanese Patent Application Laid-Open Nos. Showa 56(1981)-46234); fluorenone derivatives (Japanese Patent Application Laid-Open Nos. Showa 54(1979)-110837); hydrazone derivatives (United States Patent No. 3,717,462; and Japanese Patent Application Laid-Open Nos. Showa 54(1979)-59143, Showa 55(1980)-52063, Showa 55(1980)-52064, Showa 55(1980)-46760, Showa 55(1980)-85495, Showa 57(1982)-11350, Showa 57(1982)-148749 and Heisei 2(1990)-311591); stilbene derivatives (Japanese Patent Application Laid-Open Nos. Showa 61(1986)-210363, Showa 61(1986)-228451, Showa 61(1986)-14642, Showa 61(1986)-72255, Showa 62(1987)-47646, Showa 62(1987)-36674, Showa 62(1987)-10652, Showa 62(1987)-30255, Showa 60(1985)-93455, Showa 60(1985)-94462, Showa 60(1985)-174749 and Showa 60(1985)-175052); silazane derivatives (United States Patent No. 4,950,950); polysilane-based compounds (Japanese Patent Application Laid-Open No. Heisei 2(1990)-204996); aniline-based copolymers (Japanese Patent Application Laid-Open No. Heisei 2(1990)-282263); and electrically conductive macromolecular oligomers (in particular, thiophene oligomers) disclosed in Japanese Patent Application Laid-Open No. Heisei 1(1989)-211399.

Besides the above materials which can be used as the material for the hole injecting layer, porphyrin compounds (compounds disclosed in Japanese Patent Application Laid-Open No. Showa 63(1988)-295695); and aromatic tertiary amine compounds and styrylamine compounds (United States Patent No. 4,127,412 and Japanese Patent Application Laid-Open Nos. Showa 53(1978)-27033, Showa 54(1979)-58445, Showa 54(1979)-149634, Showa 54(1979)-64299, Showa 55(1980)-79450. Showa 55(1980)-144250, Showa 56(1981)-119132, Showa 61(1986)-295558, Showa 61(1986)-98353 and Showa 63(1988)-295695) are preferable, and the aromatic tertiary amines are more preferable.

Further examples include compounds having two condensed aromatic rings in the molecule which are described in the United States Patent No. 5,061,569 such as 4,4'-bis(N-(1-naphthyl)-N-phenylamino)-biphenyl and a compound in which three triphenylamine units are bonded together in a star-burst shape, which is described in Japanese Patent Application Laid-Open No. Heisei 4(1992)-308688, such as 4,4',4"-tris(N-(3-methylphenyl)-N-phenylamino)triphenylamine.

Besides the aromatic dime thylidene-based compounds described as the material for the light emitting layer, inorganic compounds such as Si of the p-type and SiC of the p-type can be used as the material for the hole injecting layer.

The hole injecting and transporting layer can be formed by preparing a thin film of the amine-based compound of the present invention and/or the above compound in accordance with a conventional process such as the vacuum vapor deposition process, the spin coating process, the casting process and the LB process. The thickness of the hole injecting and transporting layer is not particularly limited. In general, the thickness is 5 nm to 5 µm.

The organic semiconductor layer is a layer helping injection of holes or electrons into the light emitting layer. As the organic semiconductor layer, a layer having a conductivity of 10⁻¹⁰ S/cm or greater is preferable. As the material for the organic semiconductor layer, oligomers containing thiophene can be used, and conductive oligomers such as oligomers containing arylamine and conductive dendrimers such as dendrimers containing arylamine, which are disclosed in Japanese Patent Application Laid-Open No. Heisei 8(1996)-193191, can also be used.

The electron injecting and transporting layer is a layer which helps injection of electrons into the light emitting layer and transports electrons to the light emitting region and exhibits a great mobility of electrons.

It is known that, in an organic EL device, emitted light is reflected at an electrode (the cathode in the present case), and the light emitted and obtained directly from the anode and the light obtained after reflection at the electrode interfere with each other. The thickness of the electron transporting layer is suitably selected in the range of several nm to several µm so that the interference is effectively utilized. When the thickness is great, it is preferable that the mobility of electrons is at least 10⁻⁵ cm²/Vs or greater under the application of an electric field of 10⁴ to 10⁶ V/cm so that the increase in the voltage is prevented.

As the material used for the electron injecting layer, metal complexes of 8-hydroxyquinoline and derivatives thereof and oxadiazole derivatives are preferable. Examples of metal complexes of 8-hydroxyquinoline and the derivative thereof include metal chelated oxinoid compounds including chelate compounds of oxines (in general, 8-quinolinol or 8-hydroxy- quinoline). For example, tris(8-quinolinol)aluminum can be used as the electron injecting material.

Examples of the oxadiazole derivative include electron transfer compounds represented by the following general formulae:

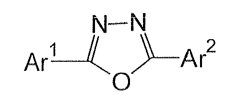

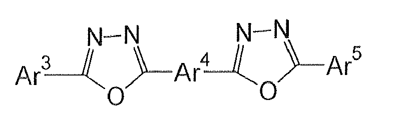

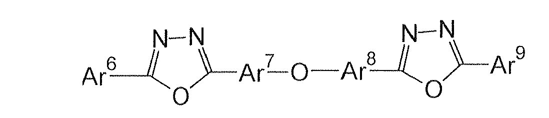

In the above formulae, Ar¹, Ar², Ar³, Ar⁵, Ar⁶ and Ar⁹ each represent a substituted or unsubstituted aryl group and may represent the same group or different groups. Ar⁴, Ar⁷ and Ar⁸ each represent a substituted or unsubstituted arylene group and may represent the same group or different groups.

Examples of the aryl group include phenyl group, biphenyl group, anthranyl group, perylenyl group and pyrenyl group. Examples of the arylene group include phenylene group, naphthylene group, biphenylene group, anthranylene group, perylenylene group and pyrenylene group. Examples of the substituent include alkyl groups having 1 to 10 carbon atoms, alkoxyl groups having 1 to 10 carbon atoms and cyano group. As the electron transfer compound, compounds which can form thin films are preferable.

Specific examples of the electron transfer compound include the following compounds:

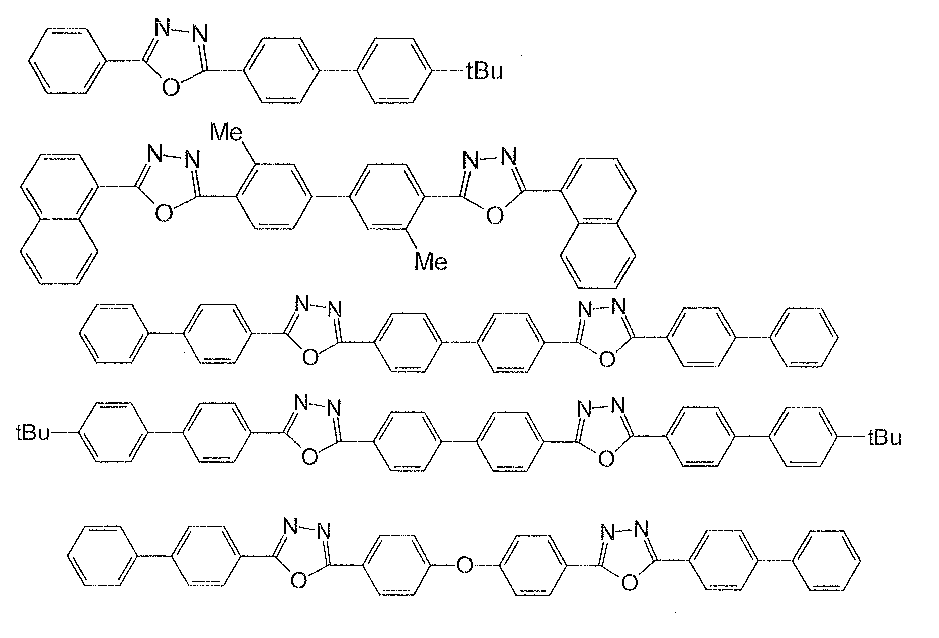

As the material which can be used for the electron injecting layer and the electron transporting layer, compounds represented by the following general formulae (A) to (E) can be used.

Heterocyclic derivatives having nitrogen atom represented by general formula (A) or (B):

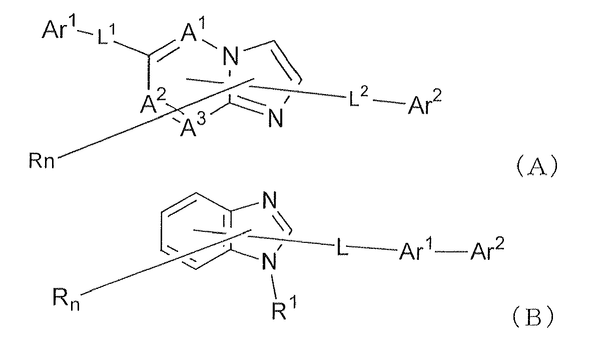

In the above general formulae (A) and (B), A¹ to A³ each independently represent nitrogen atom or carbon atom.

Ar¹ represents a substituted or unsubstituted aryl group having 6 to 60 ring carbon atoms or a substituted or unsubstituted heteroaryl group having 3 to 60 ring carbon atoms; Ar² represents hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxyl group having 1 to 20 carbon atoms or a divalent group derived from any of the above groups; and either one of Ar¹ and Ar² represents a substituted or unsubstituted condensed cyclic group having 10 to 60 ring carbon atoms or a substituted or unsubstituted monohetero condensed cyclic group having 3 to 60 ring carbon atoms.

L¹, L² and L each independently represent the single bond, a substituted or unsubstituted arylene group having 6 to 60 ring carbon atoms, a substituted or unsubstituted heteroarylene group having 3 to 60 ring carbon atoms or a substituted or unsubstituted fluorenylene group.

R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms or a substituted or unsubstituted alkoxyl group having 1 to 20 carbon atoms; n represents an integer of 0 to 5; and, when n represents an integer of 2 or greater, the atoms and the groups represented by a plurality of R may be the same with or different from each other, and a plurality of groups represented by R which are adjacent to each other may be bonded to each other to form an aliphatic ring of the carbon ring type or an aromatic ring of the carbon ring type.

Heterocyclic derivative having nitrogen atom represented by the following general formula (C):

HAr-L-Ar¹-Ar² (C)

In general formula (C), HAr represents a heterocyclic group having 3 to 40 carbon atoms and nitrogen atom which may have substituents, L represents the single bond or an arylene group having 6 to 60 carbon atoms which may have substituents, a heteroarylene group having 3 to 60 carbon atoms which may have substituents or a fluorenylene group which may have substituents, Ar¹ represents a divalent aromatic hydrocarbon group having 6 to 60 carbon atoms which may have substituents, and Ar² represents an aryl group having 6 to 60 carbon atoms which may have substituents or a heteroaryl group having 3 to 60 carbon atoms which may have substituents.

Silacyclopentadiene derivatives represented by the following general formula (D):

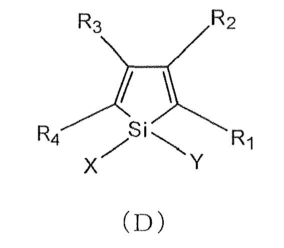

In general formula (D), X and Y each independently represent a saturated or unsaturated hydrocarbon group having 1 to 6 carbon atoms, an alkoxyl group, an alkenyloxyl group, an alkynyloxyl group, hydroxyl group, a substituted or unsubstituted aryl group, a substituted or unsubstituted heterocyclic group or a saturated or unsaturated cyclic group formed by bonding of the above groups represented by X and Y; and R₁ to R₄ each independently represent hydrogen atom, a halogen atom, a substituted or unsubstituted alkyl group having 1 to 6 carbon atoms, an alkoxyl group, an aryloxyl group, a perfluoroalkyl group, a perfluoroalkoxyl group, an amino group, an alkylcarbonyl group, an arylcarbonyl group, an alkoxycarbonyl group, an aryloxycarbonyl group, an azo group, an alkylcarbonyloxyl group, an arylcarbonyloxyl group, an alkoxycarbonyloxyl group, an aryloxycarbonyloxyl group, sulfinyl group, sulfonyl group, sulfanyl group, silyl group, carbamoyl group, an aryl group, a heterocyclic group, an alkenyl group, an alkynyl group, nitro group, formyl group, nitroso group, formyloxyl group, isocyano group, cyanate group, isocyanate group, thiocyanate group, isothiocyanate group, a cyano group or, when the groups are adjacent to each other, a structure formed by condensation of substituted or unsubstituted rings.

Borane derivatives represented by the following general formula (E):

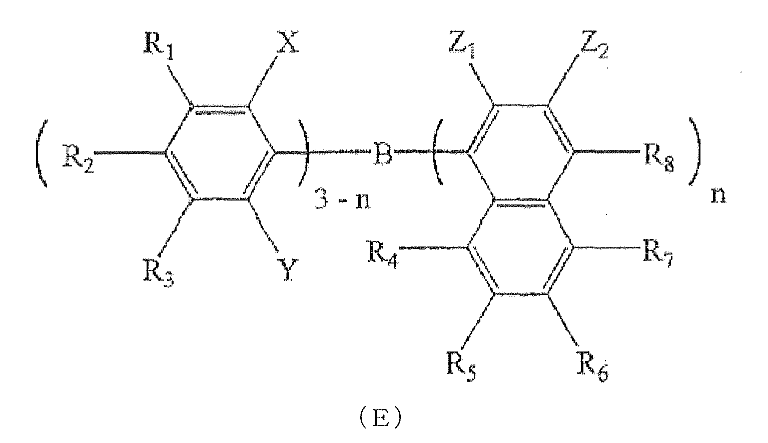

In general formula (E), R₁ to R₈ and Z₂ each independently represent hydrogen atom, a saturated or unsaturated hydrocarbon group, an aromatic group, a heterocyclic group, a substituted amino group, a substituted boryl group, an alkoxyl group or an aryloxyl group; X, Y and Z₁ each independently represent a saturated or unsaturated hydrocarbon group, an aromatic group, a heterocyclic group, a substituted amino group, an alkoxyl group or an aryloxyl group, and substituents to the groups represented by Z₁ and Z₂ may be bonded to each other to form a condensed ring; n represents an integer of 1 to 3 and, when n represents an integer of 2 or greater, a plurality of Z₁ may represent different groups; and the case where n represents 1, X, Y and R₂ each represent methyl group and R₈ represents hydrogen atom or a substituted boryl group and the case where n represents 3 and Z₁ represents methyl group are excluded.

Compounds represented by general formula (F):

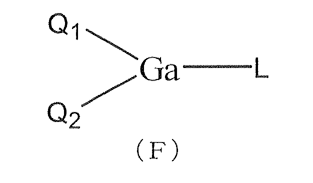

In general formula (F), Q¹ and Q² each independently represent a ligand represented by the following general formula (G): 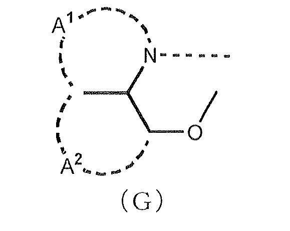 (rings A¹ and A² each representing a six-membered aryl cyclic structure which may have substituents and are condensed with each other), L represents a halogen atom, a substituted or unsubstituted alkyl group, a substituted or unsubstituted cycloalkyl group, a substituted or unsubstituted aryl group, a substituted or unsubstituted heterocyclic group, -OR¹ (R¹ representing hydrogen atom, a substituted or unsubstituted alkyl group, a substituted or unsubstituted cycloalkyl group, a substituted or unsubstituted aryl group or a substituted or unsubstituted heterocyclic group) or -O-Ga-Q³(Q⁴) (Q³ and Q⁴ are as defined for Q¹ and Q²).

The above metal complex compound strongly exhibits the property as the n-type semiconductor and a great ability of electron injection. Since the energy of formation of the complex compound is small, the bonding between the metal and the ligand in the formed metal complex compound is strong, and the quantum efficiency of fluorescence as the light emitting material is great.

Examples of the substituent to rings A¹ and A² forming the ligand represented by general formula (G) include halogen atoms such as chlorine atom, bromine atom, iodine atom and fluorine atom; substituted and unsubstituted alkyl groups such as methyl group, ethyl group, propyl group, butyl group, sec-butyl group, tert-butyl group, pentyl group, hexyl group, heptyl group, octyl group, stearyl group and trichloromethyl group; substituted and unsubstituted aryl groups such as phenyl group, naphthyl group, 3-methylphenyl group, 3-methoxyphenyl group, 3-fluorophenyl group, 3-trichloromethylphenyl group, 3-trifluoromethylphenyl group and 3-nitrophenyl group; substituted and unsubstituted alkoxyl groups such as methoxyl group, n-butoxyl group, tert-butoxyl group, trichloromethoxyl group, trifluoroethoxyl group, pentafluoropropoxyl group, 2,2,3,3-tetrafluoropropoxyl group, 1,1,1,3,3,3-hexafluoro-2-propoxyl group and 6-(perfluoroethyl)hexyloxyl group; substituted and unsubstituted aryloxyl groups such as phenoxyl group, p-nitrophenoxyl group, p-tert-butylphenoxyl group, 3-fluorophenoxyl group, pentafluorophenoxyl group and 3-trifluoromethylphenoxyl group; substituted and unsubstituted alkylthio groups such as methylthio group, ethylthio group, tert-butylthio group, hexylthio group, octylthio group and trifluoromethylthio group; substituted and unsubstituted arylthio groups such as phenylthio group, p-nitrophenylthio group, p-tert-butylphenylthio group, 3-fluorophenylthio group, pentafluorophenylthio group and 3-trifluoromethylphenylthio group; cyano group; nitro group; amino group; mono- and disubstituted amino groups such as methylamino group, diethylamino group, ethylamino group, diethylamino group, dipropylamino group, dibutyl-amino group and diphenylamino group; acylamino groups such as bis(acetoxymethyl)amino group, bis(acetoxyethyl)amino group, bis(acetoxypropyl)amino group and bis(acetoxybutyl)amino group; hydroxyl group; siloxyl group; acyl group; carbamoyl groups such as methylcarbamoyl group, dimethylcarbamoyl group, ethylcarbamoyl group, diethylcarbamoyl group, propylcarbamoyl group, butylcarbamoyl group and phenylcarbamoyl group; carboxylic acid group; sulfonic acid group; imide group; cycloalkyl groups such as cyclopentane group and cyclohexyl group; aryl groups such as phenyl group, naphthyl group, biphenyl group, anthranyl group, phenanthryl group, fluorenyl group and pyrenyl group; and heterocyclic groups such as pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triatinyl group, carbazolyl group, furanyl group, thiophenyl group, oxazolyl group, oxadiazolyl group, benzoxazolyl group, thiazolyl group, thiadiazolyl group, benzothiazolyl group, triazolyl group, imidazolyl group, benzimidazolyl group and planyl group. The above substituents may be bonded to each other to form a six-membered aryl group or a heterocyclic group.

A device comprising a reducing dopant in the interfacial region between the region transporting electrons or the cathode and the organic layer is preferable as an embodiment of the organic EL device of the present invention. The reducing dopant is defined as a substance which can reduce a compound having the electron transporting property. Various compounds can be used as the reducing dopant as long as the compounds have the specific reductive property. For example, at least one substance selected from the group consisting of alkali metals, alkaline earth metals, rare earth metals, oxides of alkali metals, halides of alkali metals, oxides of alkaline earth metals, halides of alkaline earth metals, oxides of rare earth metals, halides of rare earth metals, organic complexes of alkali metals, organic complexes of alkaline earth metals and organic complexes of rare earth metals can be advantageously used.

Preferable examples of the reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV) and Cs (the work function: 1.95 eV) and at least one alkaline earth metal selected from the group consisting of Ca (the work function: 2.9 eV), Sr (the work function: 2.0 to 2.5 eV) and Ba (the work function: 2.52 eV). Among the above substances, at least one alkali metal selected from the group consisting of K, Rb and Cs is more preferable, Rb and Cs are still more preferable, and Cs is most preferable as the reducing dopant. These alkali metals have great reducing ability, and the luminance of the emitted light and the life of the organic EL device can be increased by addition of a relatively small amount of the alkali metal into the electron injecting zone. As the reducing dopant having a work function of 2.9 eV or smaller, combinations of two or more alkali metals are also preferable. Combinations having Cs such as the combinations of Cs and Na, Cs and K, Cs and Rb and Cs, Na and K are more preferable. The reducing ability can be efficiently exhibited by the combination having Cs. The luminance of emitted light and the life of the organic EL device can be increased by adding the combination having Cs into the electron injecting zone.

In the present invention, an electron injecting layer which is constituted with an insulating material or a semiconductor may further be disposed between the cathode and the organic layer. By disposing the electron injecting layer, leak of electric current can be effectively prevented, and the electron injecting property can be improved. As the insulating material, at least one metal compound selected from the group consisting of alkali metal chalcogenides, alkaline earth metal chalcogenides, halides of alkali metals and halides of alkaline earth metals is preferable. It is preferable that the electron injecting layer is constituted with the above substance such as the alkali metal chalcogenide since the electron injecting property can be further improved. Preferable examples of the alkali metal chalcogenide include Li₂O, K₂O, Na₂S, Na₂Se and Na₂O. Preferable examples of the alkaline earth metal chalcogenide include CaO, BaO, SrO, BeO, BaS and CaSe. Preferable examples of the halide of an alkali metal include LiF, NaF, KF, LiCl, KCl and NaCl. Preferable examples of the halide of an alkaline earth metal include fluorides such as CaF₂, BaF₂, SrF₂, MgF₂ and BeF₂ and halides other than the fluorides.

Examples of the semiconductor constituting the electron transporting layer include oxides, nitrides and oxide nitrides of at least one metal selected from Ba, Ca, Sr, Yb, Al, Ga, In, Li, Na, Cd, Mg, Si, Ta, Sb and Zn used singly or in combination of two or more. It is preferable that the inorganic compound constituting the electron transporting layer forms a fine crystalline or amorphous insulating thin film. When the electron injecting layer is constituted with the insulating thin film described above, a more uniform thin film can be formed, and defects of pixels such as dark spots can be decreased. Examples of the inorganic compound include alkali metal chalcogenides, alkaline earth metal chalcogenides, halides of alkali metals and halides of alkaline earth metals which are described above.

For the cathode, a material such as a metal, an alloy, a conductive compound or a mixture of these materials which has a small work function (4 eV or smaller) is used as the electrode material. Examples of the electrode material include sodium, sodium-potassium alloys, magnesium, lithium, magnesium-silver alloys, aluminum/ aluminum oxide, Al/Li₂O, Al/LiO₂, Al/LiF, aluminum-lithium alloys, indium and rare earth metals.

The cathode can be prepared by forming a thin film of the electrode material described above in accordance with a process such as the vapor deposition process and the sputtering process.

When the light emitted from the light emitting layer is obtained through the cathode, it is preferable that the cathode has a transmittance of the emitted light greater than 10 %. It is also preferable that the sheet resistivity of the cathode is several hundred Ω/□ or smaller. The thickness of the cathode is, in general, selected in the range of 10 nm to 1 µm and preferably in the range of 50 to 200 nm.

Defects in pixels tend to be formed in organic EL device due to leak and short circuit since an electric field is applied to ultra-thin films. To prevent the formation of the defects, it is preferable that a layer of a thin film having an insulating property is inserted between the pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be used.

To prepare the organic EL device of the present invention, for example, the anode, the light emitting layer, the hole injecting layer where necessary and the electron injecting layer where necessary are formed in accordance with the above process using the above materials, and the cathode is formed in the last step. The organic EL device may be prepared by forming the above layers in the order reverse to that described above, i.e., the cathode being formed in the first step and the anode in the last step.

An embodiment of the process for preparing an organic EL device having a construction in which an anode, a hole injecting layer, a light emitting layer, an electron injecting layer and a cathode are disposed successively on a substrate transmitting light will be described in the following.

On a suitable substrate which transmits light, a thin film made of a material for the anode is formed in accordance with the vapor deposition process or the sputtering process so that the thickness of the formed thin film is 1 µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process or the LB process as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions are suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the used compound (the material for the hole injecting layer) and the crystal structure and the recombination structure of the hole injecting layer to be formed.

Then, the light emitting layer is formed on the hole injecting layer formed above. Using the light emitting material of the present invention, a thin film of the organic light emitting material can be formed in accordance with the vacuum vapor deposition process, the sputtering process, the spin coating process or the casting process, and the formed thin film is used as the light emitting layer. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer although the conditions are different depending on the used compound.

The electron injecting layer is formed on the light emitting layer formed above. Similarly to the hole injecting layer and the light emitting layer, it is preferable that the electron injecting layer is formed in accordance with the vacuum vapor deposition process since a uniform film must be obtained. The conditions of the vacuum vapor deposition can be selected in the same ranges as those described for the vacuum vapor deposition of the hole injecting layer and the light emitting layer.

In the use of the compound of the present invention, specific procedures and conditions are different depending on whether the compound is used for the light emitting zone or for the hole transporting zone. When the vacuum vapor deposition process is used, the compound of the present invention can be vapor deposited simultaneously in combination with other materials, and when the spin coating process is used, the compound of the present invention can be used in combination with other materials by mixing the materials together.

The cathode is formed on the electron injecting layer formed above in the last step, and the organic EL device can be obtained. The cathode is made of a metal and can be formed in accordance with the vacuum vapor deposition process or the sputtering process. It is preferable that the vacuum vapor deposition process is used in order to prevent formation of damages on the lower organic layers during the formation of the film.

In the above preparation of the organic EL device, it is preferable that the above layers from the anode to the cathode are formed successively while the preparation system is kept in a vacuum after being evacuated.

The process for forming the layers in the organic EL device of the present invention is not particularly limited. A conventional process such as the vacuum vapor deposition process and the spin coating process can be used. The organic thin film layer comprising the amine-based compound of the present invention can be formed in accordance with a conventional process such as the vacuum vapor deposition process and the molecular beam epitaxy process (the MBE process) or, using a solution prepared by dissolving the compounds into a solvent, in accordance with a coating process such as the dipping process, the spin coating process, the casting process, the bar coating process and the roll coating process.

The thickness of each layer in the organic thin film layer in the organic EL device of the present invention is not particularly limited. A thickness in the range of several nanometers to 1 µm is preferable so that defects such as pin holes are decreased and the efficiency can be improved.

When a direct voltage is applied to the organic EL device, emission of light can be observed under application of a voltage of 5 to 40 V in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

The present invention will be described more specifically with reference to examples in the following. However, the present invention is not limited to the examples.

### Synthesis Example 1 (Synthesis of Compound (1): 5,8-bis[{4-(diphenylamino)phenyl}phenylamino]quinoxaline)

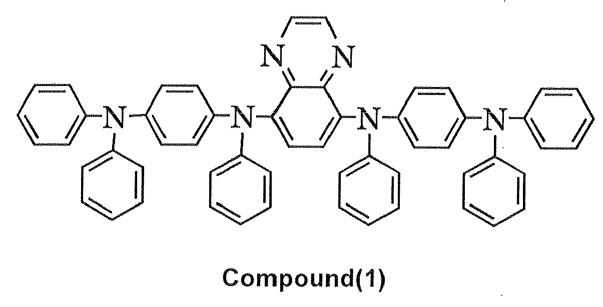

(1) Synthesis of N,N-diphenyl-N'-phenyl-p-phenylenediamine
   The reaction was conducted under the stream of argon. Into a 20 ml two-necked flask, 600 mg of molecular sieves 5A (adsorbing zeolite composed of crystalline sodium aluminosilicate; manufactured by Wako Pure Chemical Industries, Ltd.) was placed and stirred under heating under a reduced pressure sufficiently. To the above, 55 mg (the formula weight (FW): 915.70; 0.06 mmole) of Pd₂(dba)₃ (tris(dibenzylileneacetone) dipalladium(0)), 0.24 mmole (FW: 202.3; 89 µl as a 2.7 M toluene solution) of tBu₃P, 807 mg (FW: 96.11; 8.4 mmole) of Na(OtBu) and 1.946 g (FW: 324.22; 6 mmole) of 4-bromophenyldiphenylamine were added. Then, 18.2 ml of toluene as the solvent was added, and the resultant mixture was sufficiently stirred. To the resultant mixture, 615 mg (FW: 93.129; 6.6 mmole) of aniline was added, and the reaction was allowed to proceed under the refluxing condition (about 80°C) for 6 hours. The reaction product was filtered through Celite and sufficiently washed with ethyl acetate. The obtained product was purified in accordance with the silica gel column chromatography (the developing solvent: chloroform). As the result, 1.96 g (the yield: 97%) of the object product (N,N-diphenyl-N'-phenyl-p-phenylenediamine) was obtained (the Rf value: about 0.8).
   The obtained product was analyzed in accordance with the field desorption mass analysis (FD-MS), and the major ion peak was found at 336, which agreed with the calculated value. 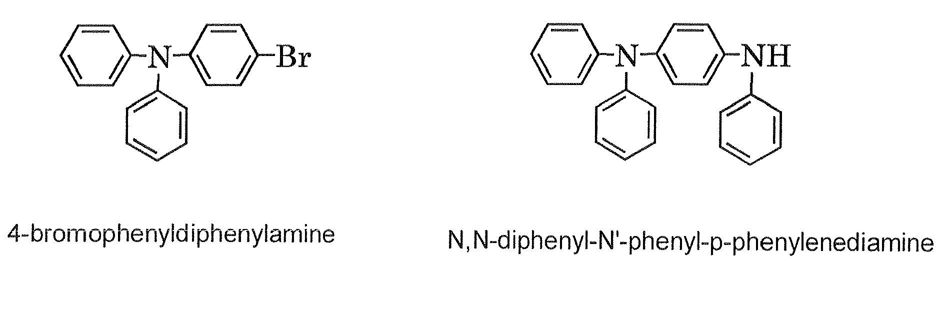
(2) Synthesis of 4,7-dibromobenzothiadiazole
   Into a 200 ml three-necked flask, 29.05 g (213 mmole) of benzothiadiazole was placed and dissolved into 42.9 ml of a 47% aqueous solution of HBr. To the obtained solution, 32.24 ml of bromine was added dropwise at the room temperature over 20 minutes, and then 21.3 ml of a 47% aqueous solution of HBr was added. The resultant mixture was heated under the refluxing condition for 24 hours. After the resultant mixture was cooled to the room temperature, the solid component was dissolved into 1100 ml of dichloromethane. To the obtained solution, 400 ml of a saturated aqueous solution of sodium thiosulfate was added, and the obtained mixture was treated by extraction sufficiently in a separation funnel. The obtained dichloromethane layer was washed three times each with 150 ml of distilled water and dried with anhydrous sodium sulfate. The dried dichloromethane solution was concentrated to 300 ml, and the recrystallization was conducted by leaving the solution standing at 4°C for 24 hours. The formed needle crystals were separated by filtration, and 18.29 g (the yield: 29%) of the object compound (4,7-dibromobenzothiadiazole) was obtained.
(3) Synthesis of 3,6-dibromo-1,2-diaminobenzene
   Into a 500 ml three-necked flask equipped with a refluxing condenser, 10.0 g (FW: 293.96; 34 mmole) of 4,7-dibromobenzothiadiazole and 400 ml of ethanol as the solvent were placed. Under cooling with ice, 24.2 g (FW: 37.83; 640 mmole) of sodium borohydride was added, and the resultant mixture was sufficiently stirred for about 2 hours. When generation of a gas (hydrogen sulfide) was not observed any more, the temperature was adjusted at the room temperature, and the reaction mixture was left standing for one night (about 14 hours). Then, the solvent was completely removed under a reduced pressure, and about 400 ml of water was added to dissolve the residue. The resultant solution was left standing for one night, and the object product was recrystallized (7.17 g). The obtained product was treated by extraction with ethyl ether three times, washed with a saturated aqueous solution of sodium chloride, dried with anhydrous sodium sulfate and filtered. After diethyl ether was removed by distillation, 0.88 g of the object compound (3,6-dibromo-1,2-diaminobenzene) was obtained. The overall yield was 82%.
(4) Synthesis of 5,8-dibromoquinoxaline
   Into a 500 ml three-necked flask, 3,6-dibromo-1,2-diaminobenzene obtained in (3) described above (FW: 287.90; 24.3 mmole; 7.0 g), 300 ml of ethanol as the solvent and 50 ml of a 40% aqueous solution of glyoxal (about 44 mmole; FW: 58.04; d=1.28) were added, and the resultant mixture was stirred for 8 hours. Recrystallization was conducted 5 times using ethanol as the solvent, and the object compound (5,8-dibromoquinoxaline) was obtained in an approximately quantitative amount.
(5) Synthesis of Compound (1)

The reaction was conducted under the stream of argon. Into a 20 ml two-necked flask, 600 mg of molecular sieves 5A was placed and stirred under heating under a reduced pressure sufficiently. To the above, 48.4 mg (FW: 915.70; 0.0528 mmole) of Pd₂(dba)₃, 0.212 mmole (FW: 202.3; 78.6 µl as a 2.7 M toluene solution) of tBu₃P, 710 mg (FW: 96.11; 7.39 mmole) of Na(OtBu) and 1.96 g (FW: 33.644; 5.82 mmole) of N,N-diphenyl-N'-phenyl-p-phenylenediamine were added. Then, 16 ml of toluene as the solvent was added, and the resultant mixture was sufficiently stirred. To the resultant mixture, 767 mg (FW: 289.94; 2.64 mmole) of 5,8-dibromoquinoxaline was added, and the reaction was allowed to proceed under the refluxing condition (about 120°C) for 6 hours. The reaction product was filtered through Celite and sufficiently washed with ethyl acetate. The obtained product was purified in accordance with the silica gel column chromatography (the developing solvent: methylene chloride). As the result, 1.74 g (the yield: 83%) of the object product (Compound (1)) was obtained (the Rf value: about 0.5).

The obtained product was analyzed in accordance with FD-MS, and the major ion peak was found at 798, which agreed with the calculated value. The glass transition temperature as measured in accordance with the differential scanning calorimetry (DSC) was 138.6°C, and the ionization potential was 5.3 eV.

The apparatuses and the conditions of the measurements used in FD-MS, DSC and the measurement of the ionization potential are shown in the following.

### <Measurement of FD-MS>

Apparatus: HX110 (manufactured by NIPPON DENSHI Co., Ltd.)

### Conditions:

- voltage of acceleration:: 8 kV
- scanning range:: m/z=50~1,500
- species of emitter:: carbon
- current of emitter :: 0 mA→2 mA/minute→40 mA (kept for 10 minutes)

### (Measurement of DSC>

Apparatus: PERKIN ELMER PYRIS 1

### Conditions:

(1) The first heating: 30°C→260°C; the rate of temperature elevation: 10°C/minute; entirely under the atmosphere of nitrogen
(2) 260°C; kept for 3 minutes
(3) 260°C→-50°C; rapid cooling at 200°C/minute
(4) -50°C; kept for 10 minutes
(5) The second heating: -50°C→260°C; the rate of temperature elevation: 10°C/minute

### <Measurement of ionization potential>

Apparatus: Photoelectron spectrometer AC-1 (manufactured by RIGAKU Co., Ltd.

Conditions: powder sample; in the air

### Example 1 (Preparation of an organic EL device using Compound (1) as the hole injecting material)

A glass substrate (manufactured by GEOMATEC Company) of 25 mm × 75 mm × 1.1 mm thickness having an ITO transparent electrode was cleaned by application of ultrasonic wave in isopropyl alcohol for 5 minutes and then by exposure to ozone generated by ultraviolet light for 30 minutes. On the surface of the cleaned substrate at the side having the transparent electrode, a solution obtained by dissolving Compound (1) in toluene was applied in accordance with the spin coating process (the thickness of the formed film: 20 nm). The formed film was sufficiently dried under a vacuum at 100°C. The dried laminate was attached to a substrate holder of a vacuum vapor deposition apparatus. To the attached laminate, the following compound HT1 for the hole transporting layer (the thickness of the formed layer: 20 nm), the following compounds EM1 and D1 for the light emitting layer (the ratio of the amounts by weight: 40:2; the thickness of the formed layer: 40 nm), the following compound Alq for the electron transporting layer (the thickness of the formed layer: 20 nm), lithium fluoride (LiF) for the electron injecting layer (the thickness of the formed layer: 10 nm) and aluminum for the cathode were laminated successively in accordance with the vacuum vapor deposition process, and an organic EL device was obtained.

When a voltage was applied to the obtained organic EL device, a luminance (L) of 85 cd/m² and a current density (J) of 0.9 mA/cm² were obtained at a voltage of 5 V, and excellent luminance/voltage property, current density/voltage property and current efficiency (L/J×0.1) (9.3 cd/A) were exhibited. The chromaticity coordinates (X, Y) were (0.18, 0.35).

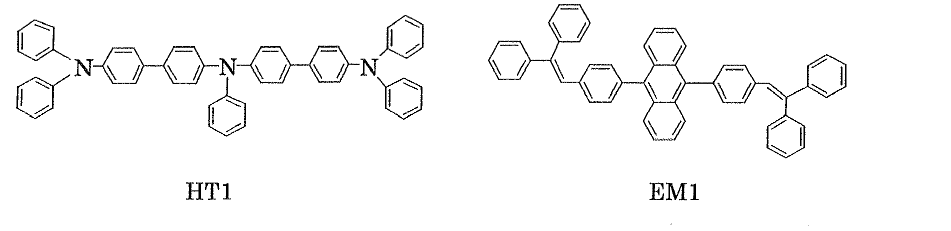

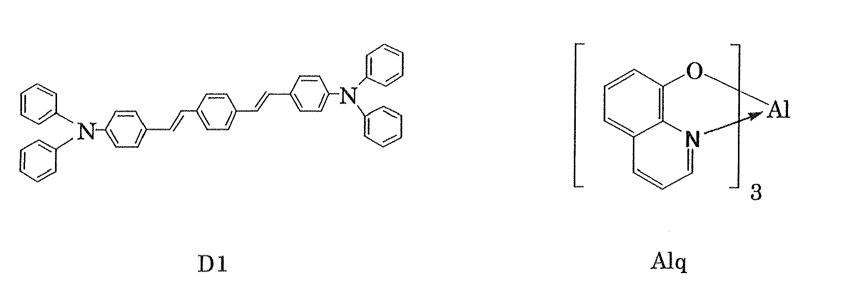

### Comparative Example 1 (Preparation of an organic EL device using PEDOT/PSS as the hole injecting material)

An organic EL device was prepared in accordance with the same procedures as those conducted in Example 1 except that, in the formation of the hole transporting layer, a solution obtained by dissolving commercial PEDOT/PSS in water was applied in accordance with the spin coating process in place of applying the solution prepared by dissolving Compound (1) in toluene in accordance with the spin coating process.

When a voltage was applied to the obtained organic EL device, a luminance (L) of 98 cd/m² and a current density (J) of 1.6 mA/cm² were obtained at a voltage of 5 V, and excellent luminance/voltage property, current density/voltage property and current efficiency (L/J×0.1) (6.1 cd/A) were exhibited. The chromaticity coordinates (X, Y) were (0.18, 0.34).

### INDUSTRIAL APPLICABILITY

As described specifically in the above, the novel amine-based compound of the present invention is suitable as the hole injecting material and the hole transporting material for photo-sensitive materials for electronic photography and organic EL devices, and a thin film can be formed in accordance with the coating process due to the excellent solubility. The organic EL device using the amine-based compound of the present invention exhibits excellent balance in physical properties such as a small ionization potential, a great band gap energy, a great efficiency of injection and a great mobility and excellent heat resistance and efficiency of light emission. Therefore, the organic EL device is highly practical, and suitable as devices used in vehicles to which heat resistance exceeding 130°C is required.

## Claims

1. An amine-based compound represented by following general formula (1) or (2): 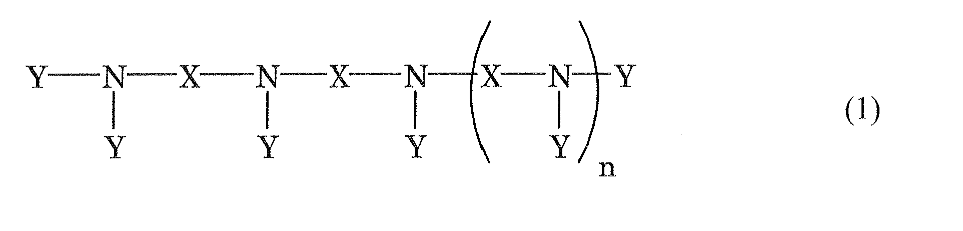 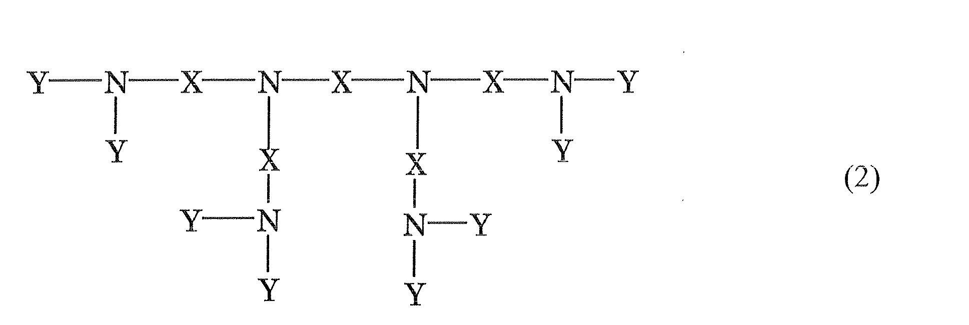 wherein X represents a substituted or unsubstituted arylene group having 4 to 50 carbon atoms or a group represented by following general formula (3) or (4), a plurality of X may represent a same group or different groups, and at least one of the plurality of X in general formulae (1) and (2) represents a group represented by one of general formulae (3) and (4);
Y represents a substituted or unsubstituted aryl group having 4 to 50 carbon atoms or a substituted or unsubstituted heterocyclic group having 5 to 50 carbon atoms, and a plurality of Y may represent a same group or different groups;
n represents an integer of 0 to 3; and N represents nitrogen atom; general formulae (3) and (4) being: 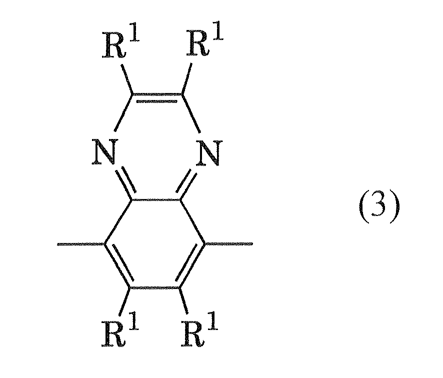 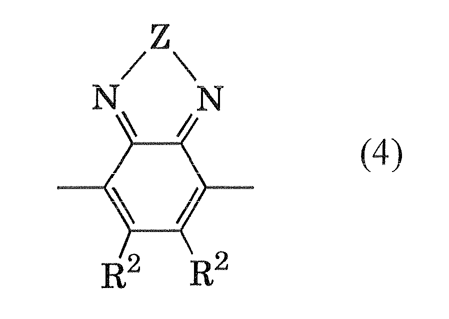 wherein R¹ and R² each independently represent hydrogen atom, a halogen atom, a substituted or unsubstituted alkyl group having 1 to 50 carbon atoms, a substituted or unsubstituted alkoxyl group having 1 to 50 carbon atoms, a substituted or unsubstituted aryloxyl group having 4 to 50 carbon atoms, a substituted or unsubstituted thioalkoxyl group having 1 to 50 carbon atoms, a substituted or unsubstituted thioaryloxyl group having 4 to 50 carbon atoms, a substituted or unsubstituted amino group, a substituted or unsubstituted aryl group having 4 to 50 carbon atoms, a substituted or unsubstituted alkylcarbonyl group having 1 to 50 carbon atoms or a substituted or unsubstituted arylcarbonyl group having 4 to 50 carbon atoms; atoms and groups represented by R¹ and R² may be same with or different from each other; and adjacent groups represented by R¹ may be bonded to each other to form a cyclic structure, and adjacent groups represented by R² may be bonded to each other to form a cyclic structure; and
Z represents sulfur atom, oxygen atom or -NR³-, R³ representing hydrogen atom, a substituted or unsubstituted aryl group having 4 to 50 carbon atoms, a substituted or unsubstituted heterocyclic group having 5 to 50 carbon atoms or a substituted or unsubstituted alkyl group having 1 to 50 carbon atoms.

2. An amine-based compound according to Claim 1, wherein n represents 1 in general formula (1).

3. An amine-based compound according to any one of Claims 1 and 2, which is a hole injecting material or a hole transporting material.

4. An amine-based compound according to any one of Claims 1 and 2, which is a hole injecting material or a hole transporting material for organic electroluminescence devices.

5. An organic electroluminescence device which comprises a cathode, an anode and an organic thin film layer which comprises at least one layer comprising at least a light emitting layer and is disposed between the cathode and the anode, wherein at least one layer in the organic thin film layer comprises the amine-based compound described in any one of Claims 1 and 2 singly or as a component of a mixture.

6. An organic electroluminescence device according to Claim 5, wherein the organic thin film layer comprises at least one of a hole injecting layer and a hole transporting layer, and at least one of the hole injecting layer and the hole transporting layer comprises the amine-based compound described in any one of Claims 1 and 2 singly or as a component of a mixture.

7. An organic electroluminescence device according to Claim 5, wherein the light emitting layer emits bluish light.

8. An organic electroluminescence device according to Claim 5, wherein the light emitting layer comprises an anthracene derivative as a host material.

9. An organic electroluminescence device according to any one of Claims 7 and 8, wherein the light emitting layer comprises an amine derivative as a host material or a dopant.

10. An amine-based compound represented by any one of following general formula (1) and (2): 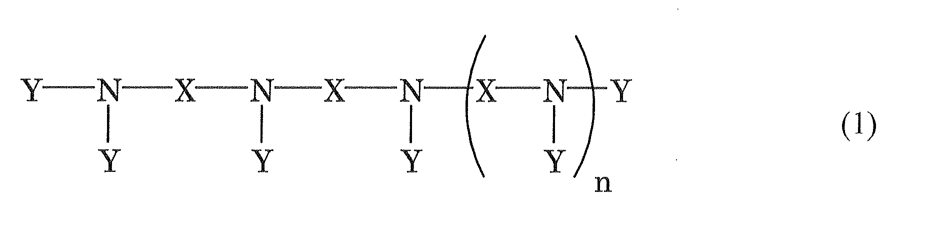 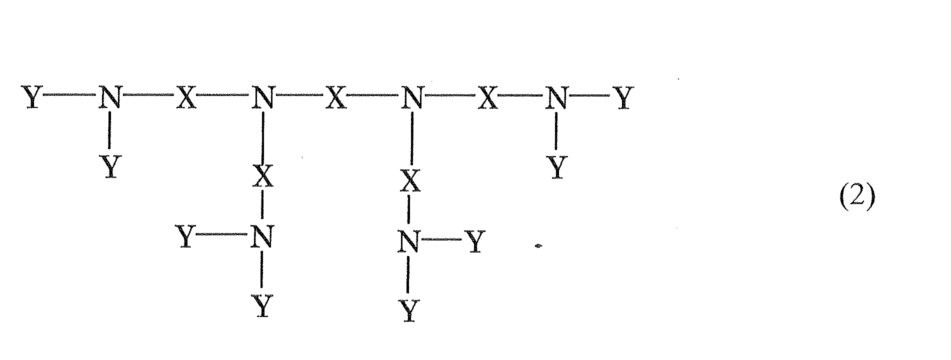 wherein X represents a heterocyclic crosslinking group, and a plurality of X may represent a same group or different groups;
Y represents a substituted or unsubstituted aryl group having 4 to 50 carbon atoms or a substituted or unsubstituted heterocyclic group having 5 to 50, and a plurality of Y may represent a same group or different groups;
n represents an integer of 0 to 3; and N represents nitrogen atom.

11. An amine-based compound according to Claim 10, wherein n represents 1 in general formula (1).

12. An amine-based compound according to any one of Claims 10 and 11, which is a hole injecting material or a hole transporting material.

13. An amine-based compound according to any one of Claims 10 and 11, which is a hole injecting material or a hole transporting material for electroluminescence devices.

14. An organic electroluminescence device which comprises a cathode, an anode and an organic thin film layer which comprises at least one layer comprising at least a light emitting layer and is disposed between the cathode and the anode, wherein at least one layer in the organic thin film layer comprises the amine-based compound described in any one of Claims 10 and 11 singly or as a component of a mixture.

15. An organic electroluminescence device according to Claim 14, wherein the organic thin film layer comprises at least one of a hole injecting layer and a hole transporting layer, and at least one of the hole injecting layer and the hole transporting layer comprises the amine-based compound described in any one of Claims 10 and 11 singly or as a component of a mixture.

16. An organic electroluminescence device according to Claim 14, wherein the light emitting layer emits bluish light.

17. An organic electroluminescence device according to Claim 14, wherein the light emitting layer comprises an anthracene derivative as a host material.

18. An organic electroluminescence device according to any one of Claims 16 and 17, wherein the light emitting layer comprises an amine derivative as a host material or a dopant.
